# EUROPEAN PATENT APPLICATION

(11) **EP 1 650 267 A1**
(43) Date of publication of application: **26.04.2006**
(21) Application number: 04735802.3
(22) Date of filing: 02.06.2004
(51) Int. Cl.: C09B 29/15, C07D 277/62, C09D 11/02, C09D 7/12, C09D 201/00, G02F 1/1335

(54) **MONOAZO COMPOUND AND METHOD FOR PRODUCING SAME**

(30) Priority: 03.06.2003 JP 2003157946
(71) Applicant: Kabushiki Kaisha Ueno Seiyaku Oyo Kenkyujo, Osaka-shi, Osaka 541-0043 (JP)
(72) Inventor: UENO, Ryuzo, Nishinomiya-shi, Hyogo 6620038 (JP); OTANI, Junji, Kobe-shi, Hyogo 657-0016 (JP); YAMASHITA, Tetsuya, Kobe-shi, Hyogo 6511321 (JP); HISANO, Takaya, 11, Ueno Seiyaku Dainishikoryo, Nishinomiya-shi, Hyogo 6620066 (JP)
(74) Representative: Albrecht, Thomas
(86) International application number: PCT/JP2004/007994
(87) International publication number: WO 2004/108833

(57) **Abstract**

The present invention provides a monoazo compound represented by formula (1) or a salt thereof: 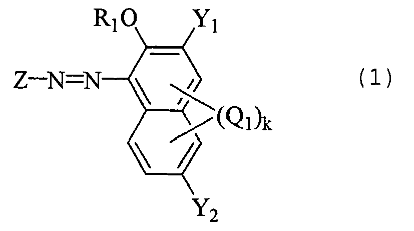 wherein, Y₁ and Y₂ represent a group selected from hydrogen atom, formula (2) and formula (3);

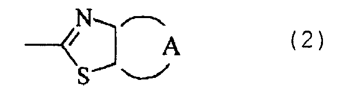

-CO-E-X (3)

provided that at least one of Y₁ and Y₂ is a group represented by formula (2);
Z is a group selected from formulae (4), (5) and (6);

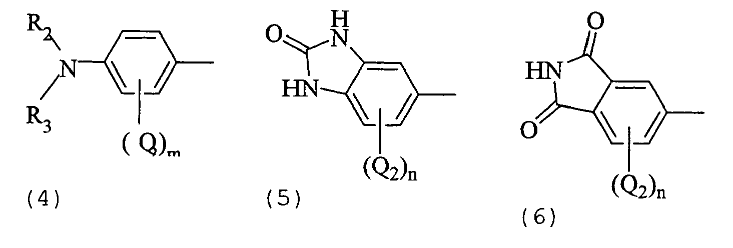

## Description

### TECHNICAL FIELD

The present invention relates to a novel monoazo compound which provides black color and exhibits high tinting strength.

### BACKGROUND OF THE INVENTION

As a black pigment, carbon black, which is one of inorganic pigments, is currently most commonly used. Organic pigments such as aniline black and perylene black are also used for certain purposes. A black matrix, which is used for various displays such as liquid crystal displays (LCDs), is one of recent important applications of these black pigments.

In manufacturing liquid crystal displays, black matrices is provided as light shielding filters between the color filters to prevent leakage of light which pass the red, green and blue filters and to improve contrast.

In addition, in thin-film transistor (TFT) displays, black matrices are formed to prevent light deterioration of the TFT properties.

Black pigments used for black matrices need to have high shielding properties (i.e. high optical densities) and low electrical conductivity.

Conventionally, chromiun metals have been commonly used for black matrices. However, chromiun metals are poisonous to human bodies and therefore, chrome-free materials have been desired. With respect to carbon black, there is a problem owing to its high electrical conductivity, that is, errors due to short-circuiting between electrodes have frequently occurred.

Aniline black is an organic black pigment with low electrical conductivity, but it is not suitable for black matrix because of its low optical density. In addition, the process for preparing aniline black involves chrome or cupper as catalyst, and such poisonous metals are inevitably remained in the pigment. Moreover, aniline black exhibits weaker coloring property than that of carbon black when used for ink, paint and the like.

Azo compounds are widely used as organic pigments and an azo compound which is prepared using a naphthol derivative having a heterocyclic group such as benzothiazolyl group, which is used in the present invention as coupler, is known (See, for example, Japanese Patent Application Laid Open (Kokai) Nos. S60-189759, S62-174768, S63-97667 and S63-293551).

All the azo compounds disclosed in the above publications are bisazo compounds or derivatives thereof and used as charge generating materials.

Though some monoazo compounds prepared by using naphthol derivatives having heterocyclic group such as benzothiazolyl group as couplers are known, compounds which are prepared by coupling such couplers with the diazonium salts used in the present invention are not known. In addition, no black color monoazo compound has been obtained. The excellent properties of such compounds as color materials such as ink, paint and dye or the use of such compounds for black matrices have not been known (See, for example, WO01/87859).

### SUMMARY OF THE INVENTION

An object of the present invention is to provide a novel black color monoazo compound with low electrical conductivity and high tinting strength.

Another object of the present invention is to provide a pigment, a print ink, a paint, a dye and a resist ink comprising the monoazo compound.

Further object of the present invention is to provide a black matrix manufactured by using the resist ink.

The present invention provides a monoazo compound represented by formula (1): 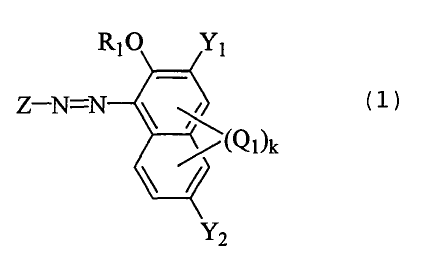 wherein Y₁ and Y₂ are selected from the group consisting of hydrogen atom, a group represented by formula (2) and a group represented by formula (3) provided that at least one of Y₁ and Y₂ represents a group represented by formula (2);

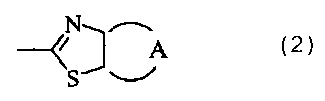

-CO-E-X (3)

wherein A is an optionally substituted aromatic group or an optionally substituted heterocyclic group having conjugated double bonds;
E is selected from -O- and -NH-;
X is selected from the group consisting of hydrogen atom, an optionally branched and optionally substituted, saturated or unsaturated aliphatic group having 1-20 carbon atoms, an optionally substituted aromatic group and an optionally substituted heterocyclic group having conjugated double bonds;
Qi is a group selected from the group consisting of an optionally branched lower alkyl group, an optionally branched lower alkoxy group, halogen atom, nitro group and nitroso group;
k is an integer of 0-3;
R₁ is selected from the group consisting of hydrogen atom, an alkaline metal, an optionally substituted and optionally branched alkyl group having 1-20 carbon atoms, an optionally substituted and optionally branched acyl group having 1-20 carbon atoms and phenylalkyl group;
Z is selected from the group consisting of a group represented by formula (4), a group represented by formula (5) and a group represented by formula (6); 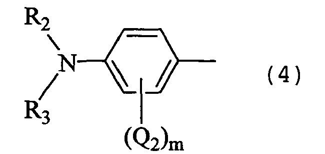 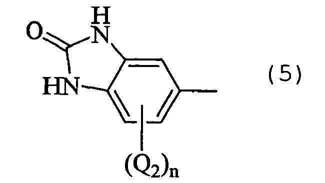 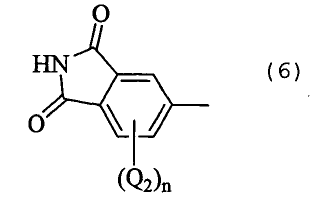 wherein Q₂ is selected from the group consisting of halogen atom, halogenated lower alkyl group, nitro group, lower alkyl group, lower alkoxy group, cyano group, phenoxy group, amino group, pyrimidylamino group, benzoylamino group, sulfonic group, hydroxy group, lower alkylaminosulfonyl group, lower alkoxycarbonyl group, phenoxycarbonyl group, lower alkylaminocarbonyl group, phenylaminocarbonyl group and alkenyl group having 2-6 carbon atoms; in which when Q₂ has an aromatic group, said aromatic group may have further substituent(s) selected from halogen atom, lower alkyl group, lower alkoxy group, phenyl group and cyano group;
m is an integer of 0-4;
n is an integer of 0-3;
R₂ and R₃ may be different or the same and are selected from the group consisting of hydrogen atom, an optionally branched and optionally substituted lower alkyl group, lower aliphatic acyl group, an optionally substituted benzoyl group and an optionally substituted phenyl group; in which R₂ or R₃ has an aromatic group, said aromatic group may have further substituent(s) selected from halogen atom, lower alkyl group, lower alkoxy group, phenyl group and cyano group;
or a salt thereof.

In the monoazo compound of the present invention represented by formula (1) , at least one of Y₁ and Y₂ is a group represented by formula (2). In the monoazo compound of the present invention where one of Y₁ and Y₂ is a group represented by formula (2), the other group is selected from hydrogen atom, a group represented by formula (2) or a group represented by formula (3). Examples of groups represented by formula (3) include carboxyl group, esterified carboxyl group (such as phenoxycarbonyl group and alkoxycarbonyl group), and amidated carboxyl group (such as alkylaminocarbonyl group, naphthylaminocarbonyl group and phenylaminocarbonyl group). Aromatic group, heterocyclic group and aliphatic group contained in groups represented by formula (2) and formula (3) may have further substituents such as halogen atom, halogenated lower alkyl group, nitro group, lower alkyl group, lower alkoxy group and cyano group.

With regard to the above formula (2), examples of optionally substituted aromatic groups include benzene ring, naphthalene ring and anthraquinone ring. Examples of optionally substituted heterocyclic groups having conjugated double bonds include thiophene, furan, pyrrole, imidazole, pyrazole, isothiazole, isooxazole, pyridine, pyrazine, pyrimidine, pyridazine, triazole, tetrazole, indole, 1H-indazole, purine, 4H-quinolizin, isoquinoline, quinoline, phthalazine, naphthyridine, quinoxaline, quinazoline, cinnoline, pteridine and benzofuran.

Examples of substituents on a group represented by formula (2) include halogen atom, halogenated lower alkyl group, nitro group, lower alkyl group, lower alkoxy group (such as methoxy group), cyano group, phenoxy group, amino group, pyrimidylamino group, benzoylamino group, sulfonic group, hydroxy group, esterified carboxyl group (such as alkoxycarbonyl group and phenoxycarbonyl group), amidated carboxyl group (such as phenylaminocarbonyl group), alkylaminosulfonyl group, and alkenyl group having 2 to 6 carbon atoms which may have aryl group. When such substituents contain aromatic group(s), the aromatic group may have one or more further substituents, such as, halogen atom, lower alkyl group, lower alkoxy group, phenyl group and cyano group.

The naphthalene moiety of the monoazo compound of the present invention represented by formula (1) may have Q₁ as substituent(s). Examples of Q₁ include an optionally branched alkyl group having 1 to 6 carbon atoms, an optionally branched alkoxy group having 1 to 6 carbon atoms, halogen atom, nitro group and nitroso group. The number of substituent(s) Q₁, i.e. k is typically 0 but it may be up to 3.

In the specification, the term "lower" represents a group having 1-6 carbon atoms.

"Aromatic group" represents a 6-membered monocyclic group or a condensed ring group consisting of up to 4 of aromatic rings.

"Heterocyclic group having conjugated double bonds" represents a 5- or 6-membered monocyclic group or a condensed ring group having at least one hetero atom selected from N, S and O and conjugated double bonds. When it constitutes a condensed ring group, said group may have up to 6 rings.

The present invention also provides a process for preparing the monoazo compound represented by formula (1), comprising coupling a coupler represented by formula (7) with a diazonium salt obtained by diazotizing an aniline compound selected from formula (8), formula (9) and formula (10) : 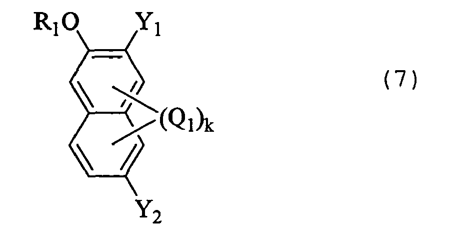 wherein Y₁, Y₂, Q₁, k and R₁ are the same as defined above. 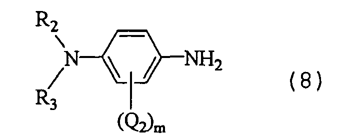 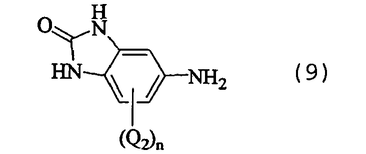 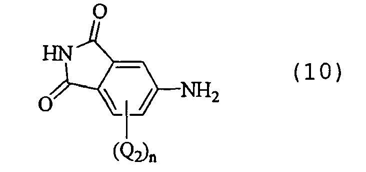 wherein Q₂, m, n, R₂ and R₃ are the same as defined above.

2-Naphthol derivative of formula (7) having a heterocyclic group is used as a coupler of the monoazo compound of the present invention and may be prepared by any known method. For example, a method disclosed in WO01/87859, which is described below, may be employed.

A hydroxynaphthalenecarboxylic acid compound such as 2-hydroxynaphthalen-3,6-dicarboxylic acid is suspended in sulfolane and the mixture is reacted with thionyl chloride. Excess thionyl chloride is then removed from the reaction. Thereafter, a compound which leads to formation of the desired heterocyclic group, for example, 2-aminothiophenol is added to the reaction. Then, phosphorus trichloride or phosphorous oxychloride is added to the mixture and the mixture is reacted at 50-180°C, preferably 50-140°C. The reaction mixture is then poured into water and the insoluble component is collected by filtration to give 2-naphthol derivative having a heterocyclic group represented by formula (7).

Alternatively, a compound to which heterocyclic group is introduced, for example, 2-hydroxynaphthalene-3,6-dicarboxylic acid or 2-aminothiophenol and sulfolane are mixed at room temperature to give a suspension, and phosphorus trichloride or phosphorous oxychloride is added dropwise to the suspension. The reaction is kept at 100°C for one hour and then heated to 140°C for one hour to give a 2-naphthol derivative having a heterocyclic group represented by formula (7).

Hydroxynaphthalenecarboxylic acids such as 2-hydroxynaphthalene-3,6-dicarboxylic acid used herein may be prepared by any known method. For example, a method comprising the steps of reacting a dehydrated alkaline metal salt of β-naphthol and carbon dioxide in the presence of solvents such as light oil, separating the resulting hydroxynaphthalenecarboxylic acid by acid crystallization and when desired, purifying the resulting compound, may be employed.

Examples of anilines represented by formulae (8), (9) and (10), which are used as diazo components in the process for preparing a monoazo compound of the present invention, include 4-N,N-dimethylaminoaniline, 4-N,N-diethylaminoaniline, 4-anilinoaniline, 4-amino-4'-methoxydiphenylamine-5-aminophthalimide, 5-aminobenzimidazolone, 4-benzoylamino-2,5-diethoxyaniline, 4-benzoylamino-2,5-dimethoxyaniline and 4-benzoylamino-5-methoxy-2-methylaniline. Among the above, 4-N,N-dimethylaminoaniline, 4-anilinoaniline, 5-aminophthalimide, and 5-aminobenzimidazolone are especially preferable.

In the method for preparing a monoazo compound of the present invention, coupling reaction of a diazo component and a coupler component is not limited, and any known method for preparing an azo compound may be employed.

A coupler component represented by formula (7) is dissolved in water or an inert organic solvent which does not interfere with coupling reaction and the solution is made basic with sodium hydroxide or the like and subjected to coupling reaction.

A diazo component may be prepared by diazotizing an aniline compound represented by formula (8), (9) or (10) with sodium nitrite or the like in an acidic solution according to the well known procedure. Thus obtained solution containing the diazonium salt may be directly subjected to the coupling reaction. Alternatively, the diazonium salt may be crystallized with fluoroboric acid or zinc chloride, collected as stable crystal and thus obtained solid crystal may be subjected to coupling reaction.

The monoazo compound of the present invention may be obtained by coupling reaction, i.e. mixing a diazonium salt, which may be present in solution or as crystal, with a coupler component solution. This coupling reaction is usually conducted at temperatures from -5°C to 100°C for 1-24 hours.

After the coupling reaction, the monoazo compound is precipitated by cooling, adding water or the like and is collected by a conventional method such as suction filtration and filter press. The resulting press cake of the monoazo compound is processed for intended uses after drying or as it is.

Another method for preparing a monoazo compound of the present invention comprises coupling a hydroxynaphthalenecarboxylic acid derivative such as 2-hydroxynaphthalene-3,6-dicarboxylic acid with a suitable diazo component and reacting thus obtained compound with a 2-aminothiophenol derivative to give a heterocyclic group on 3-and/or 6-position(s).

The monoazo compound or salt thereof obtained as above may be, if desired, purified by the method which comprises suspending the monoazo compound or salt thereof in an organic solvent selected from alcohols and aprotic polar organic solvents and stirring the mixture for 1-20 hours, preferably 3-5 hours at temperatures of 70-150°C. The purification may be carried out until no residual coupler is detected by high-performance liquid chromatography. The amount of the organic solvent may be 8-50 parts by weight, preferably 8-30 parts by weight per one part by weight of the monoazo compound.

The purification step improves purity of the monoazo compound and facilitates maturation of the crystal. The purified compound exhibits excellent crystal properties and improved resistances to light, weather or the like compared to the unpurified monoazo compound.

Therefore, the present invention provides a method for purifying a monoazo compound or a salt thereof of the present invention as well as the monoazo compound or salt thereof purified by the above method.

Examples of organic solvents used for the purifying method include alcohols such as methanol, 2-propanol and n-butanol as well as aprotic polar organic solvents such as N,N-dimethylformamide, N,N-dimethylacetamide, dimethylsulfoxide, pyridine, and N-methyl-2-pyrrolidone. Among these organic solvents, N,N-dimethylformamide, dimethylsulfoxide, and N-methyl-2-pyrrolidone are especially preferable.

In addition, when used as pigment, the monoazo compound of the present invention may be subjected to physical treatment such as kneading and milling in order to control the particle size or to miniaturize the particle.

Thus obtained monoazo compound of the present invention is black color. In the specification, the term "black color" includes both a "black" defined by chromatics and a dark color recognized as black or nearly black by eye observation. The monoazo compound of the present invention is suitably used for pigments applied to electrophotographic toners, pigment solid solutions, ink jet inks or aqueous or oily print inks for common printings, aqueous or oily paints for automobiles or building materials, dies for cellulose fibers, and resist inks for manufacturing black matrices for color filters of displays.

The pigment comprising the monoazo compound of the present invention may be produced by any known method. For example, surface of the monoazo compound particle of the present invention may be treated with a rosin, a surfactants or the like, and thus treated particles may be admixed with dispersants, humectants or the like. Examples of rosins suitably used are wood rosin, gum rosin, tall oil rosin, hydrogenated rosin, rosin ester, disproportionated rosin and polymerized rosin. Surfactants may be anionic, cationic or nonionic surfactants.

The pigment comprising the monoazo compound of the present invention may suitably be used as a black colorant for electrophotographic toners of electrostatic copiers or laser beam printers. Such toners may be manufactured by molten kneading the mixture of the pigment comprising the monoazo compound of the present invention and fixing resins according to conventional method, cooling and crushing the mixture and dispersing the pigments. Other additives such as charge control material, mold lubricant or the like may be also admixed.

The print ink comprising the monoazo compound of the present invention may be produced by the same method as that of known print ink. Examples of components contained in the print ink of the present invention include offset ink varnishes, for example, aqueous varnishes, vegetable oily varnishes such as linseed oil and synthetic resin varnishes such as phenol resin, gravure ink varnishes, for example, natural resin varnishes such as rosins, semisynthetic resin varnishes such as nitrocellulose, cellulose acetate, synthetic resin varnishes such as polyamide resin, acrylic resin, polyurethane, and polyester.

The paint comprising the monoazo compound of the present invention may be prepared by the same method as that of known paint. Examples of components contained in the paint of the present invention include one or more resin components selected from alkyd resin, acrylic resin, urethane resin, polyester resin and the like as well as one or more solvent components selected from water, butanol, isopropanol, ethyl acetate, toluene, xylene, methylethylketone and the like.

The dye comprising the monoazo compound of the present invention may be prepared by the same method as that of known dye. Examples of components contained in the dye of the present invention include dispersant, humectant, antifoaming agent and buffering agent. These components may be admixed with the monoazo compound according to conventional method.

The resist ink comprising the monoazo compound of the present invention may be prepared by the same method as that of known resist ink. The resist ink of the present invention may be negative or positive. Examples of components contained in the resist ink of the present invention include photo polymerization initiator, compounds having unsaturated double bonds which allow addition condensation, binder resin and solvent.

In order to form black matrix on a display board using the resist ink of the present invention, any known method may be employed. For example, the resist ink is applied on the display board and then solvent contained in the ink is removed by heating. Thereafter, the black matrix pattern on the display board is exposed to light and then developed, washed and baked to give a black matrix.

The novel monoazo compound of the present invention is black color and exhibits low electrical conductivity and high tinting strength.

### BRIEF DESCRIPTION OF THE DRAWINGS

Fig.1 shows an infrared absorption spectrum of the compound of formula (I).
Fig. 2 shows an infrared absorption spectrum of the compound of formula (II).
Fig. 3 shows an infrared absorption spectrum of the compound of formula (III).
Fig.4 shows an infrared absorption spectrum of the compound of formula (IV).
Fig.5 shows an infrared absorption spectrum of the compound of formula (V).
Fig.6 shows an infrared absorption spectrum of the compound of formula (VI).
Fig.7 shows an infrared absorption spectrum of the compound of formula (VII).
Fig.8 shows an infrared absorption spectrum of the compound of formula (VIII).
Fig.9 shows an infrared absorption spectrum of the compound of formula (IX).
Fig.10 shows an infrared absorption spectrum of the compound of formula (X).
Fig.11 shows an infrared absorption spectrum of the compound of formula (XI).
Fig.12 shows an infrared absorption spectrum of the compound of formula (XII).
Fig.13 shows an infrared absorption spectrum of the compound of formula (XIII).
Fig.14 shows an infrared absorption spectrum of the compound of formula (XIV).
Fig. 15 shows an infrared absorption spectrum of the compound of formula (XV).
Fig. 16 shows an infrared absorption spectrum of the compound of formula (XVI).
Fig.17 shows an infrared absorption spectrum of the compound of formula (XVII).
Fig.18 shows an infrared absorption spectrum of the compound of formula (XVIII).
Fig. 19 shows an infrared absorption spectrum of the compound of formula (XIX).

### EXAMPLES

The present invention will be further illustrated by the following examples.

### Example 1

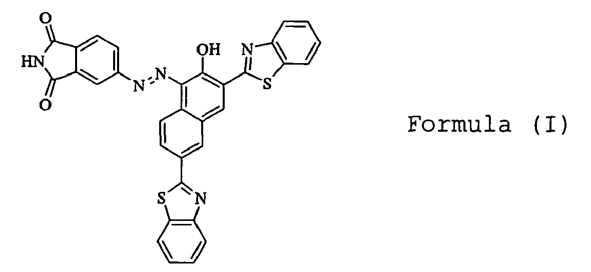

### Preparation of diazonium salt

15 g of 5-aminophthalimide was added to 105 g of water and to this mixture, 39 g of a 35% aqueous hydrochloric acid was added dropwise. The reaction was kept at temperatures no higher than 5°C and 48.7 g of a 16% aqueous sodium nitrite was added dropwise. Thereafter, the reaction was kept at temperatures from -2 to 2°C for 120 minutes with stirring and the insoluble matter was removed by suction filtration to give an aqueous 5-diazophthalimide chloride.

The resulting aqueous solution was kept at 5°C and to this solution, 39 g of a 42% aqueous fluoroboric acid was added dropwise under stirring. The precipitate was collected by suction filtration and washed with isopropyl alcohol to yield 22.7 g of 5-diazophthalimide tetrafluoroborate.

### Preparation of coupler solution

To 48 g of N-methyl-2-pyrrolidone, 6.0 g (14.6 mmols) of 2-hydroxy-3,6-bis-(1',3'-benzothiazol-2'-yl)naphthalene and 1.3 g of a 98% aqueous sodium hydroxide were added. Then, the reaction is mixed to give a coupler solution.

### Diazocoupling reaction

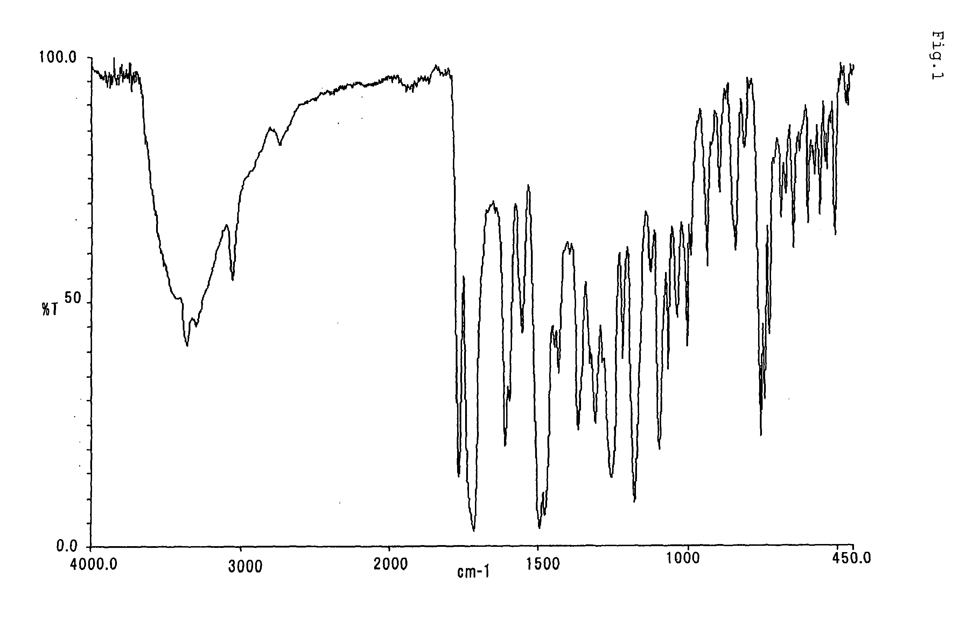

4.6 g (17.6 mmols) of 5-diazophthalimide tetrafluoroborate, the molar amount of which was 1.2-times the coupler component, was added to the coupler solution at temperatures no higher than 8°C. The diazocoupling reaction was conducted by stirring the mixture for 12 hours. To the resulting solution, 2.1 g of acetic acid was added and the mixture was stirred for 120 minutes. The precipitate was collected by suction filtration and dispersed in and washed with water and methanol, dried to give 4.6 g of the monoazo compound of formula (I) as black color powders. The infrared absorption spectrum (KBr method) of this compound is shown in Fig.1.

### Purification

One part by weight of the obtained monoazo compound was dispersed in ten parts by weight of N,N-dimethylformamide and the suspension was heated to 120°C and stirred for three hours at this temperature. After cooled to room temperature, the precipitate was collected by filtration, washed thoroughly with methanol and dried with 120°C oven. Thus purified precipitate was crushed with blender.

### Examples 2-11

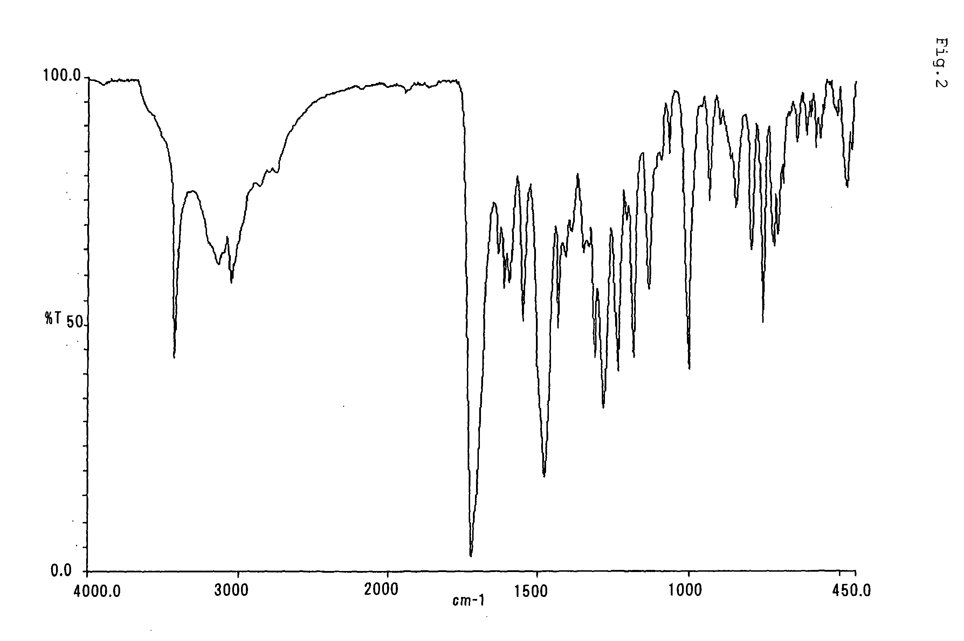

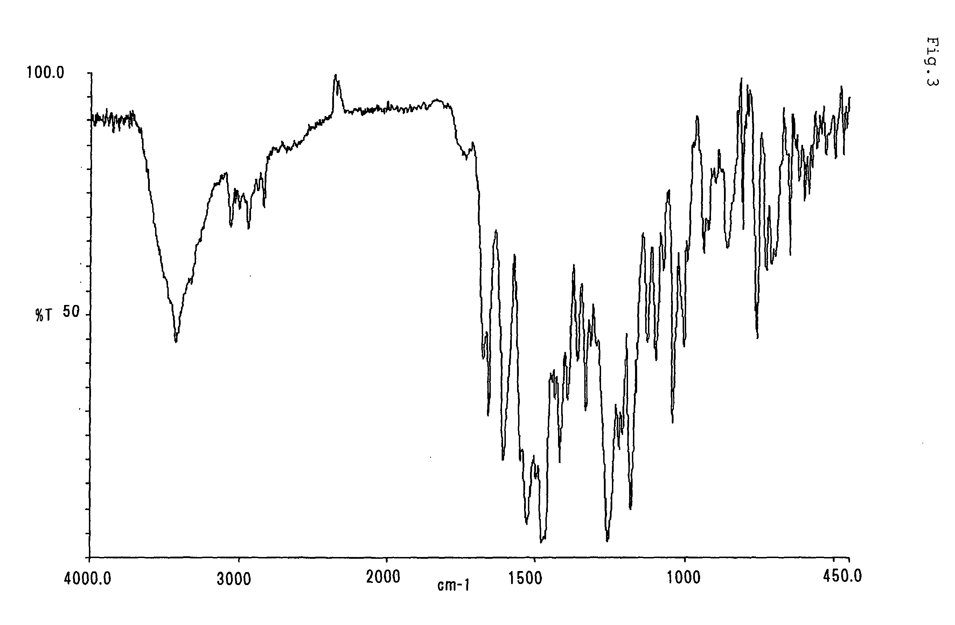

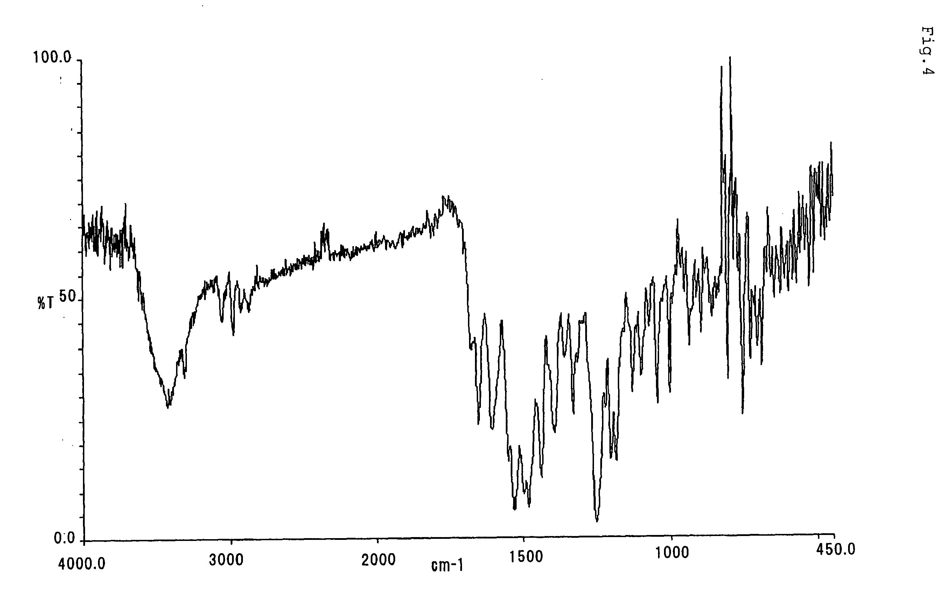

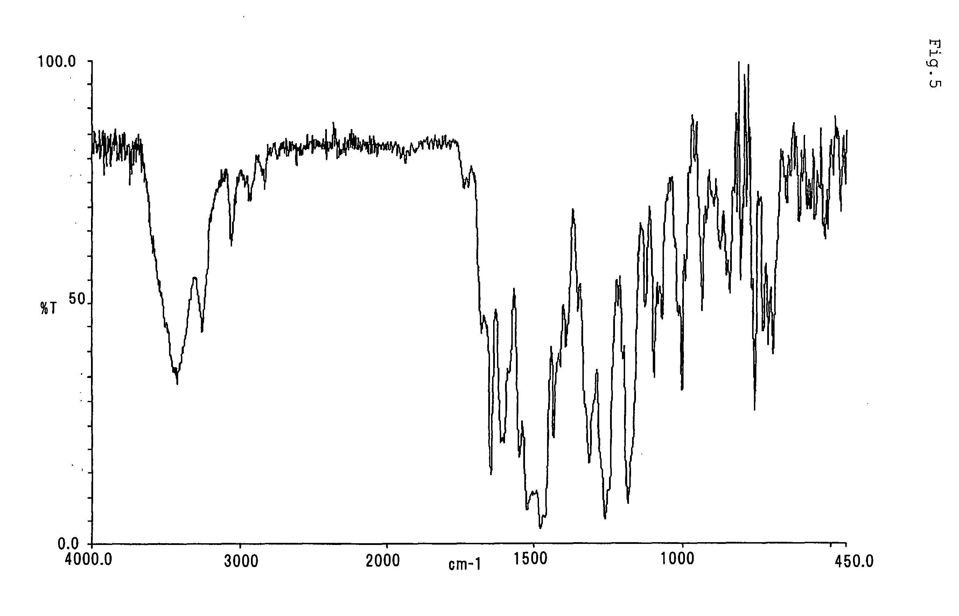

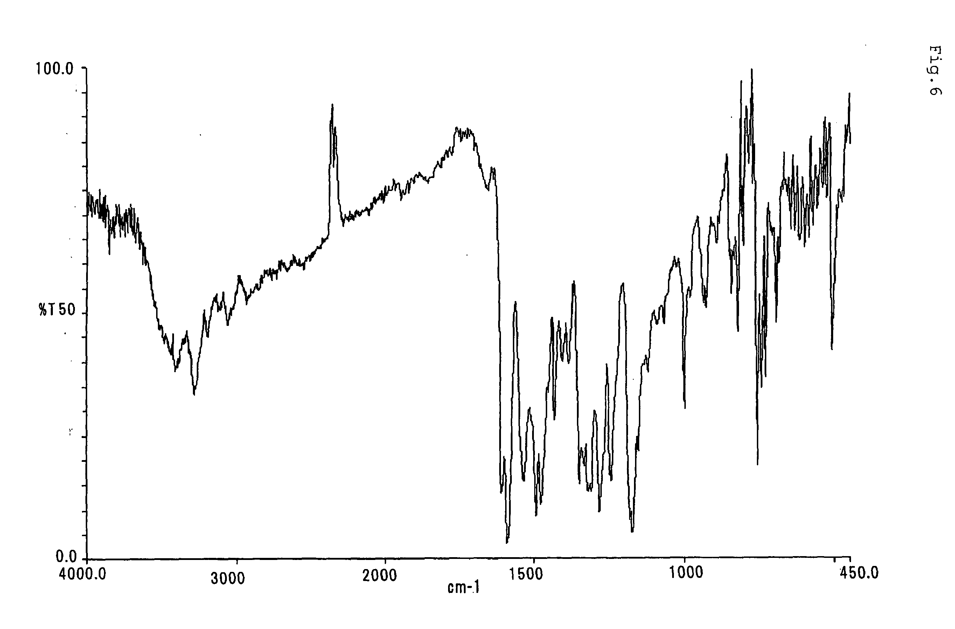

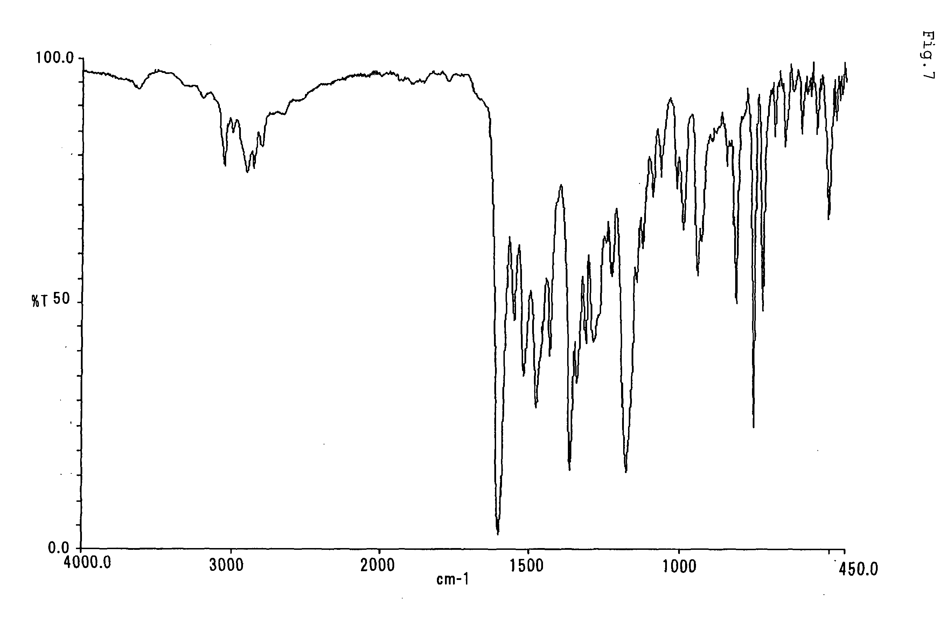

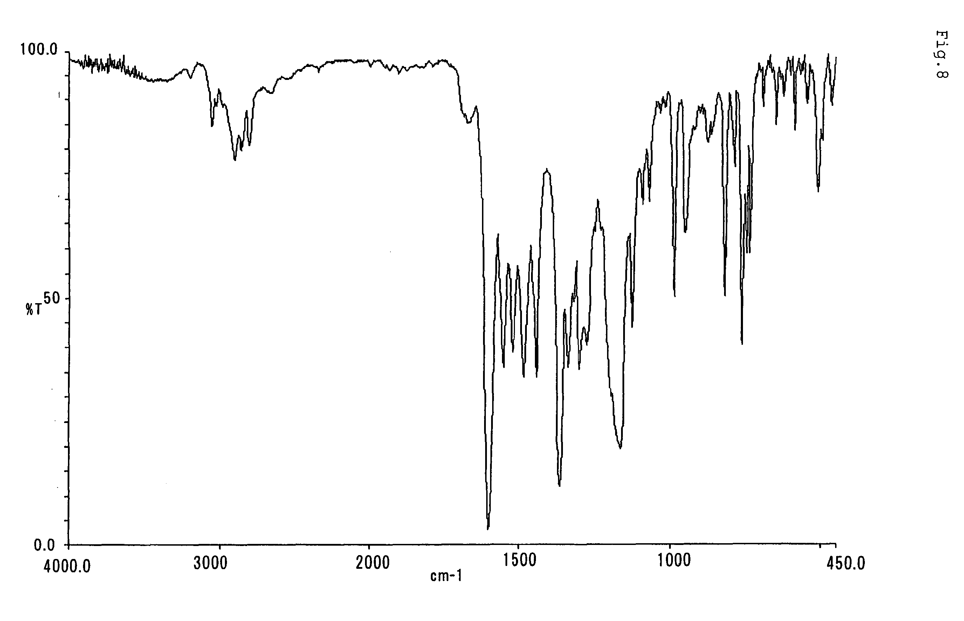

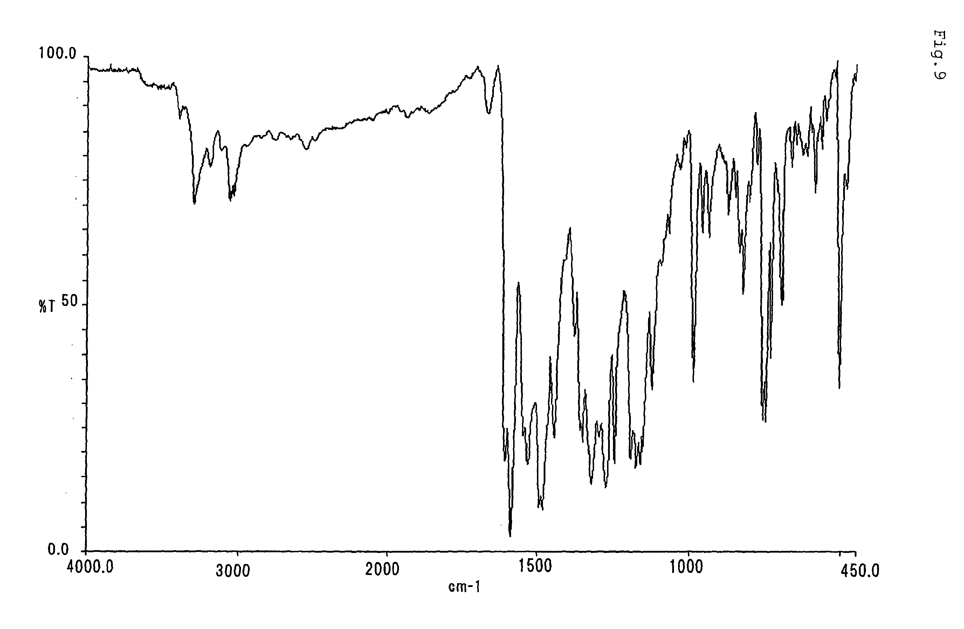

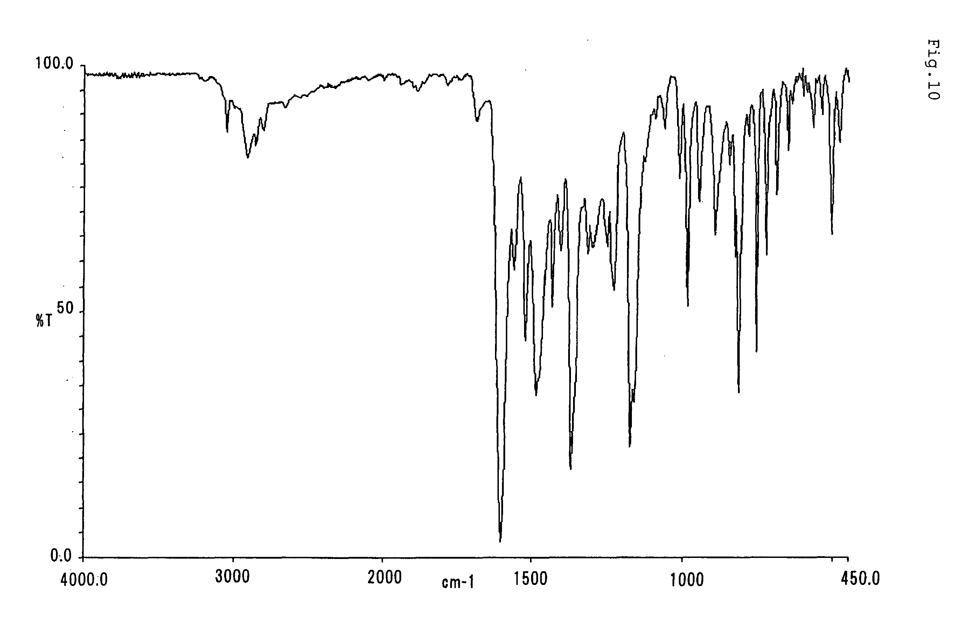

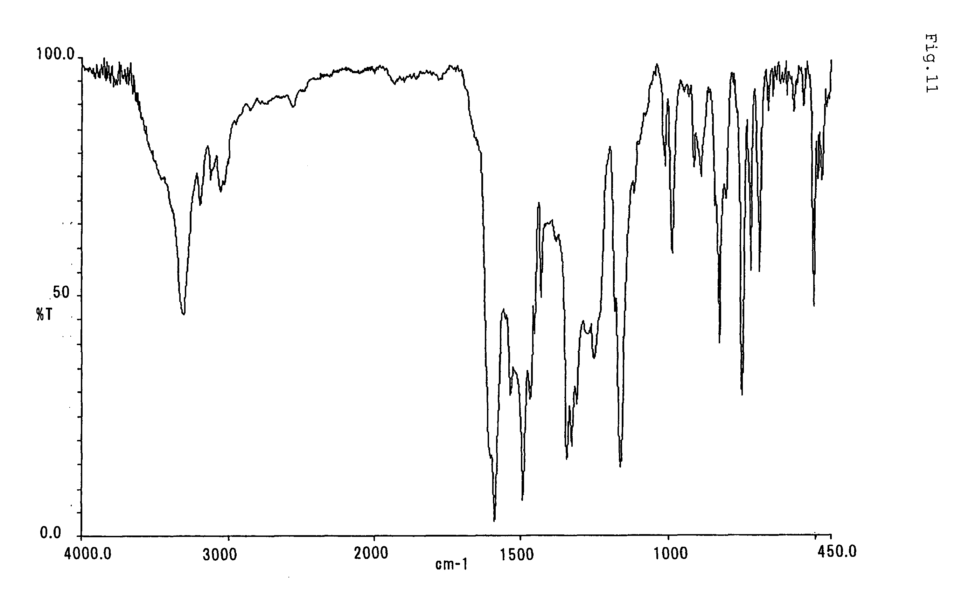

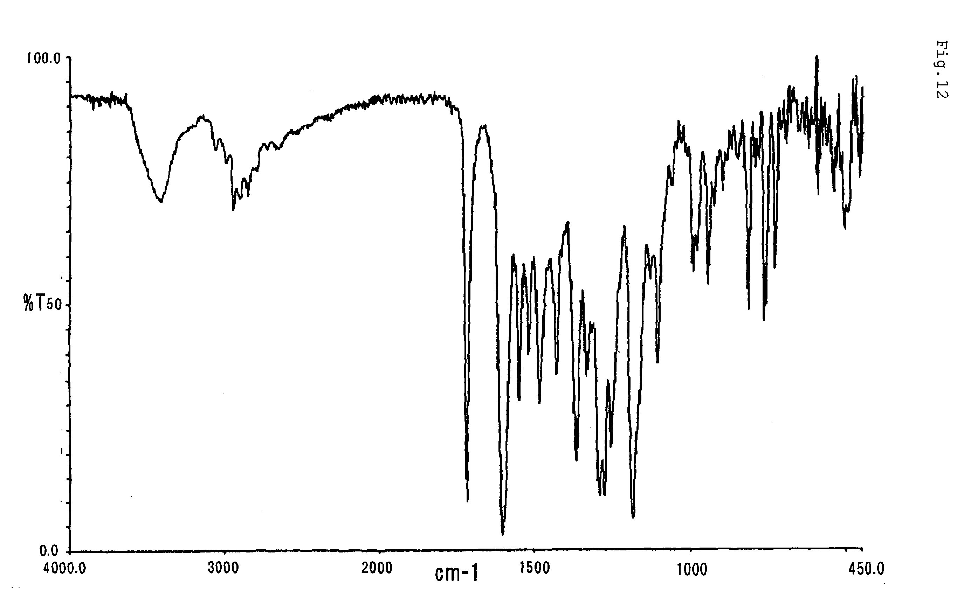

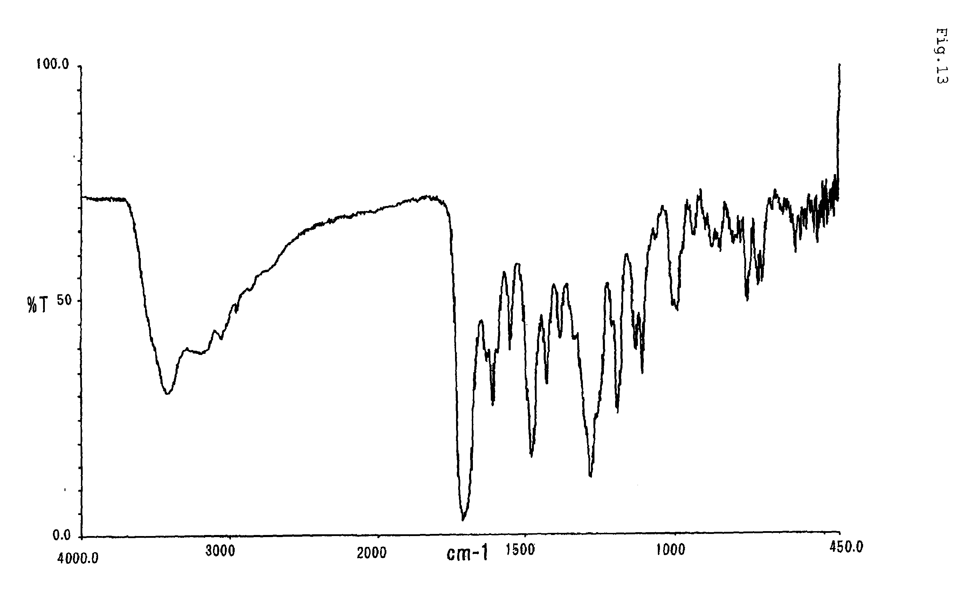

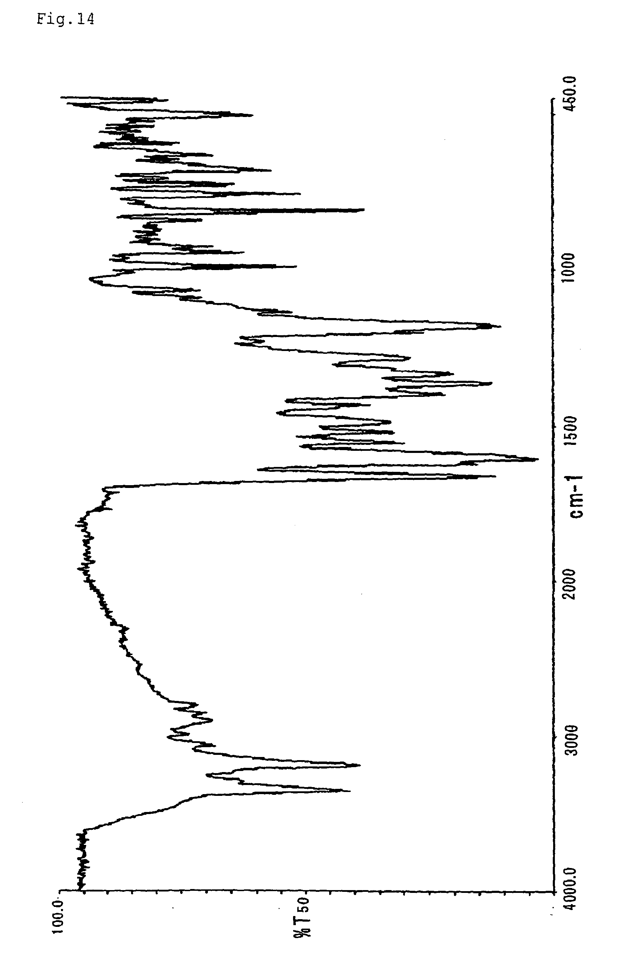

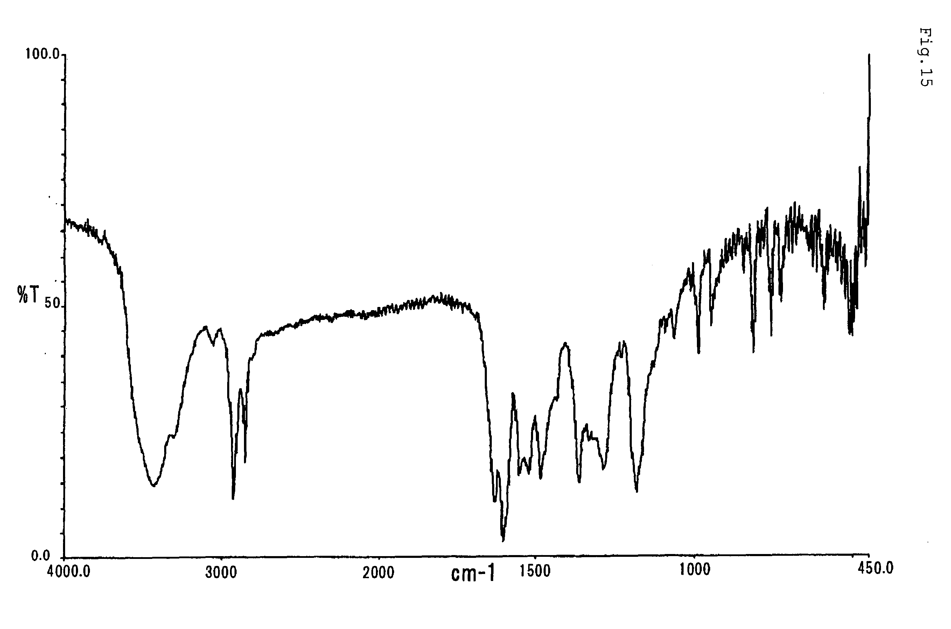

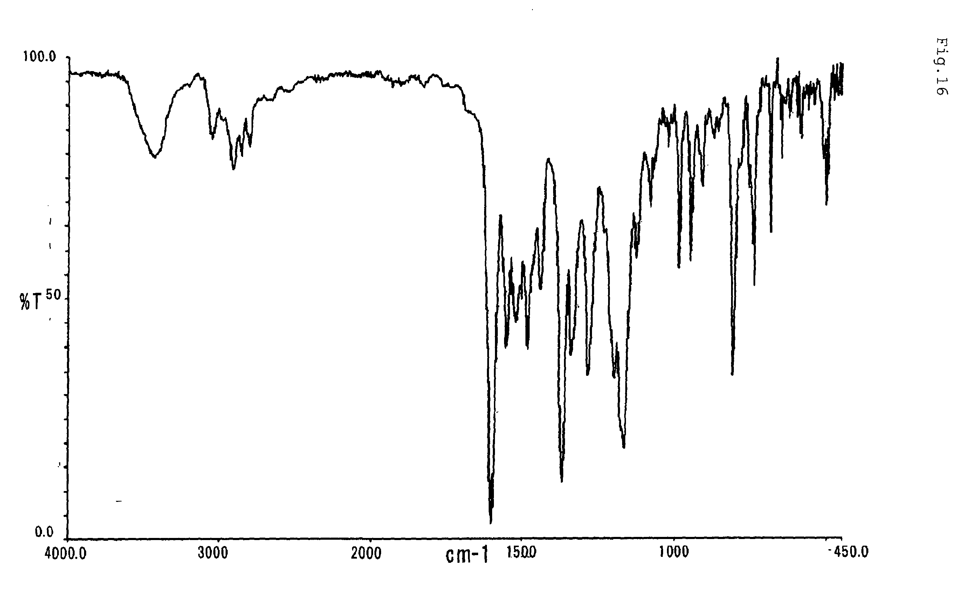

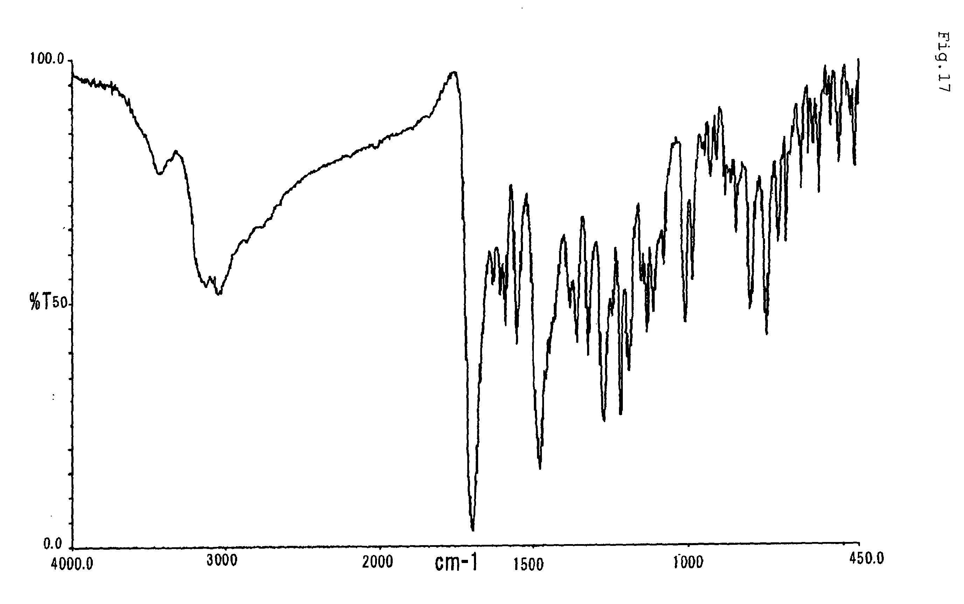

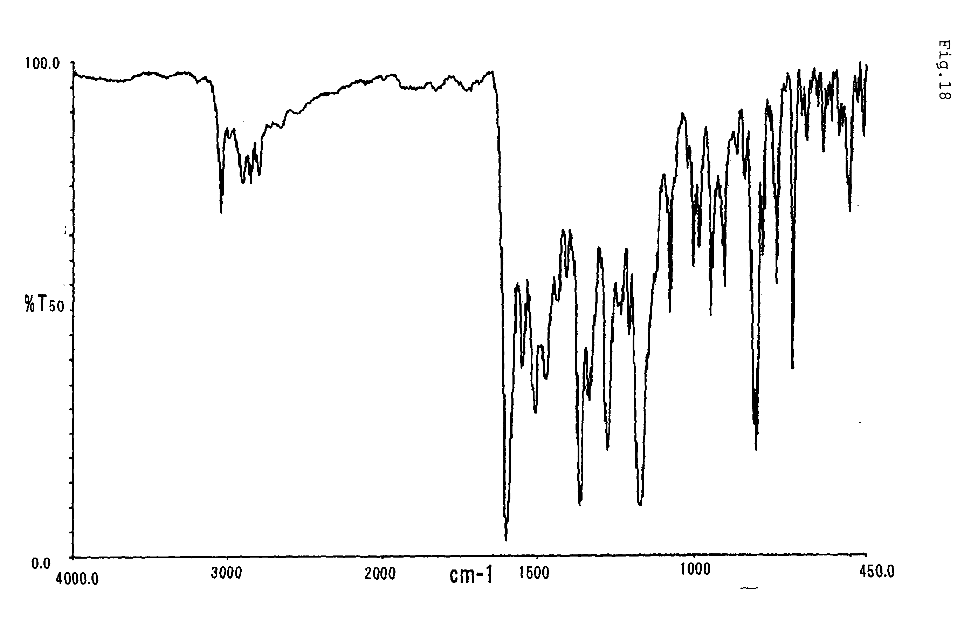

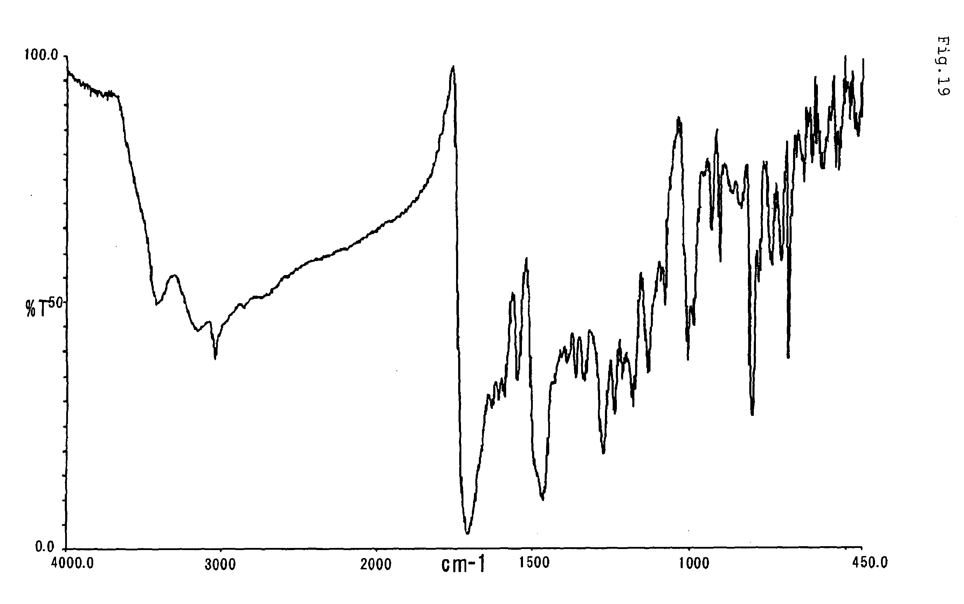

Compounds represented by formulae (II)-(XIX) were synthesized and purified according to the same manner as Example 1 with the exception that diazonium salts and couplers shown in Table 1 were used. The infrared absorption spectra (KBr method) of them are shown in Figs.2-19.

In these examples, the compounds of Examples 12, 14, and 16-18 were purified by using organic solvents of which weights were eight times those of the monoazo compounds and the compound of Example 15 was purified by using an organic solvent of which weight was 12 times those of the monoazo compound. In addition, the compounds of Examples 17 and 18 were purified using N-methyl-2-pyrrolidone as organic solvent instead of N,N-dimethylformamide.

In case 4-diazo-N,N-dimethylanilinechloride zinc chloride was used as a diazonium salt, it was purchased from Tokyo Kasei Kogyo Co., Ltd.

Synthetic examples of 2-hydroxy-3-[1',3'-(1",2"-naphthothiazole)-2'-yl]naphthalenewhich was used as couplers in Examples 16 and 17 as well as those of 2-hydroxy-3,6-bis[1',3'-(1",2" -naphthothiazole)-2'-yl] naphthalene which was used as couplers in Examples 18 and 19 were described below.

### Synthetic example 1

### 2-hydroxy-3-[1',3'-(1",2"-naphthothiazole)-2'-yl]naphthalene

### Synthesis of 1-amino-2-naphthalenethiol

1-Aminonaphthalenethiol was synthesized by the method disclosed in US Patent No.4808580.

To 60 ml of thionyl chloride, 14.5 g of 1-(1-naphthyl)-2-thiourea was added while the temperature was kept at 30-40°C. To this mixture, 30 ml of thionyl chloride was added and the reaction was stirred at temperatures of 50-55°C for 1.5 hours. After cooling the reaction to room temperature, 100 ml of ethyl acetate was added to the reaction. The resulting precipitates were collected by suction filtration, dried under reduced pressure to yield 14 g of naphtho[1,2-d]thiazole-2-amine.

To 20 g of a 98% solution of sodium hydroxide in 20 ml of water and 90ml of ethylene glycol, 9.0 g of naphtho[1,2-d]thiazole-2-amine was added and refluxed under nitrogen flow for 20 hours. The solution was diluted with 50 ml of water and then cooled to room temperature. The reaction was extracted with ether and water layer was neutralized with acetic acid. After the neutralization, water layer was extracted again with ether and the resulting ether layer was washed with water, and dried with magnesium sulfate. After removal of magnesium sulfate, ether was distilled out under reduced pressure to yield 5.5 g of 1-amino-2-naphthalenethiol.

### Synthesis of [2-hydroxy-3-[1',3'-(1'',2''-naphthothiazole)-2'-yl]naphthalene

5.0 g of 2-hydroxy-3-naphthoic acid, 5.5 g of 1-amino-2-naphthalenethiol and 4.4 g of phosphorus trichloride were added to 30 g of sulfolane and the mixture was stirred at a 140°C for three hours. After cooling the reaction to room temperature, the precipitates were collected by suction filtration. Thus collected precipitates were dispersed in and washed with methanol, and dried to yield 5.7 g of 2-hydroxy-3-[1',3'-(1",2"-naphthothiazole)-2'-yl]naphthalene.

### Synthetic example 2

### Synthesis of [2-hydroxy-3,6-bis[1',3'-(1",2"-naphthothiazole)-2'-yl]naphthalene

5.0 g of 2-hydroxynaphthalene-3,6-dicarboxylic acid, 8.0 g of 1-amino-2-naphthalenethiol and 5.9 g of phosphorus trichloride were added to 110 g of sulfolane and the mixture was stirred at a temperature of 140°C for three hours. After cooling the reaction to room temperature, methanol was added to the reaction. The precipitates were collected by suction filtration. The collected precipitates were dispersed in and washed with N-methyl-2-pyrrolidone, washed thoroughly with methanol and dried to yield 4. 1 g of 2-hydroxy-3,6-bis[1',3'-(1",2"-naphthothiazole)-2'-yl]naphthalene.

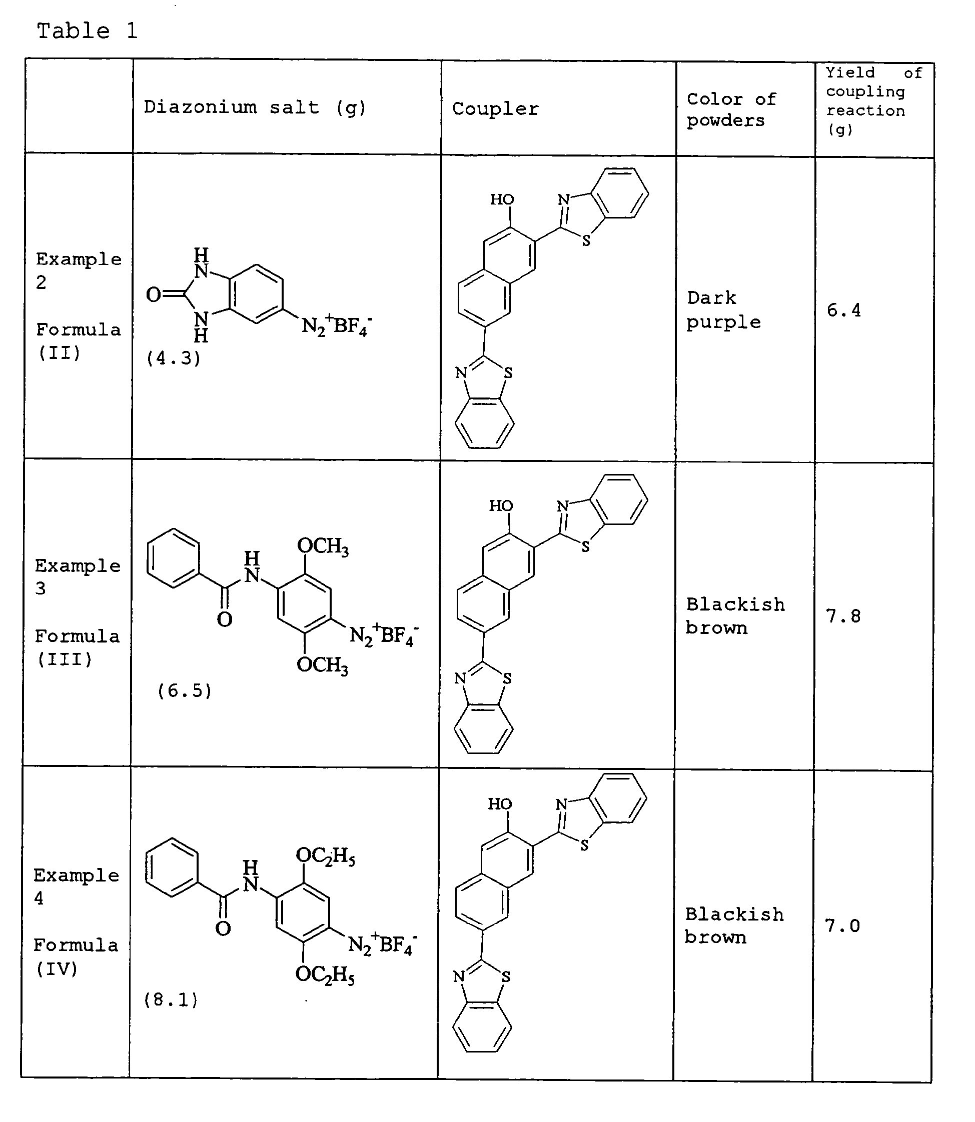

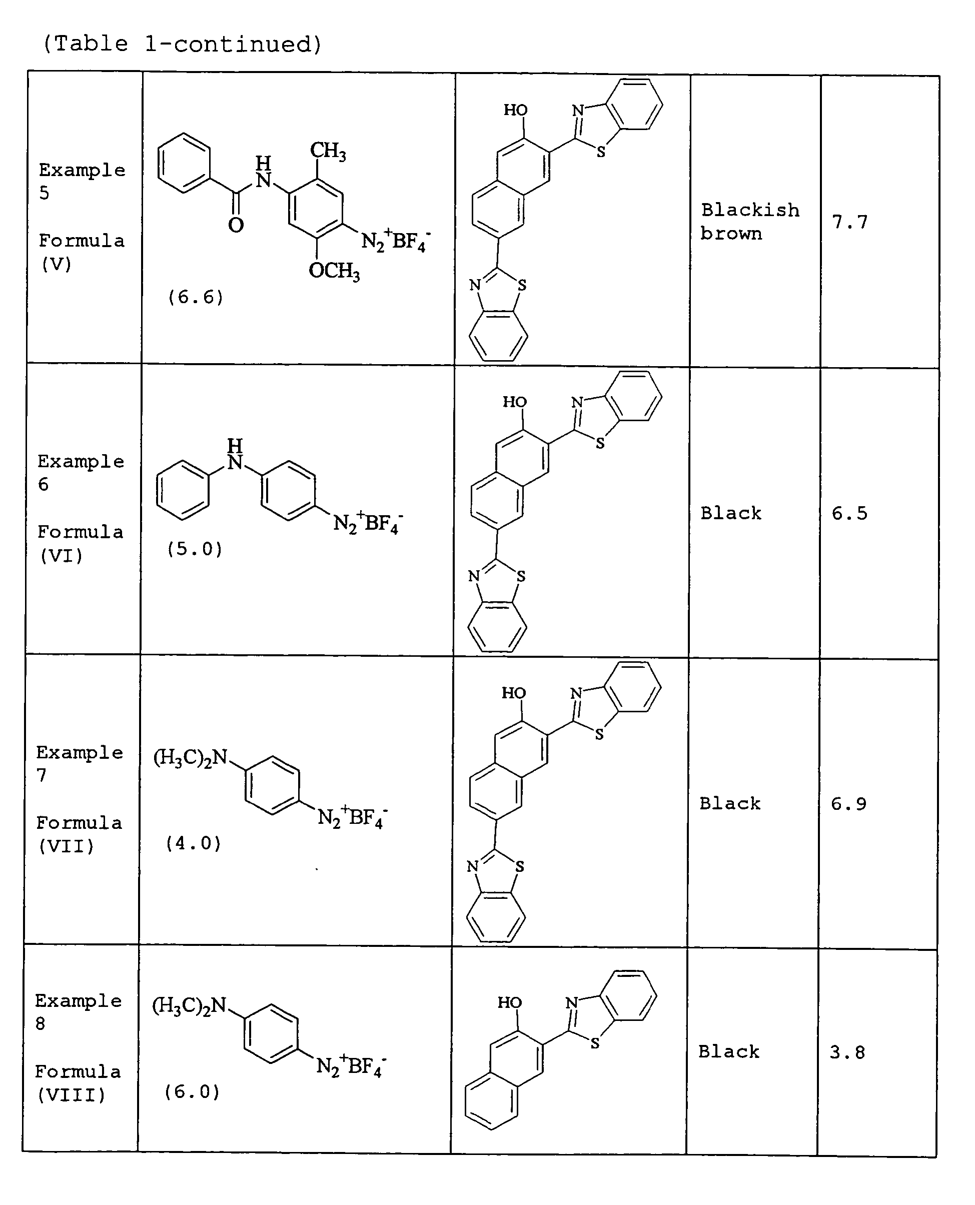

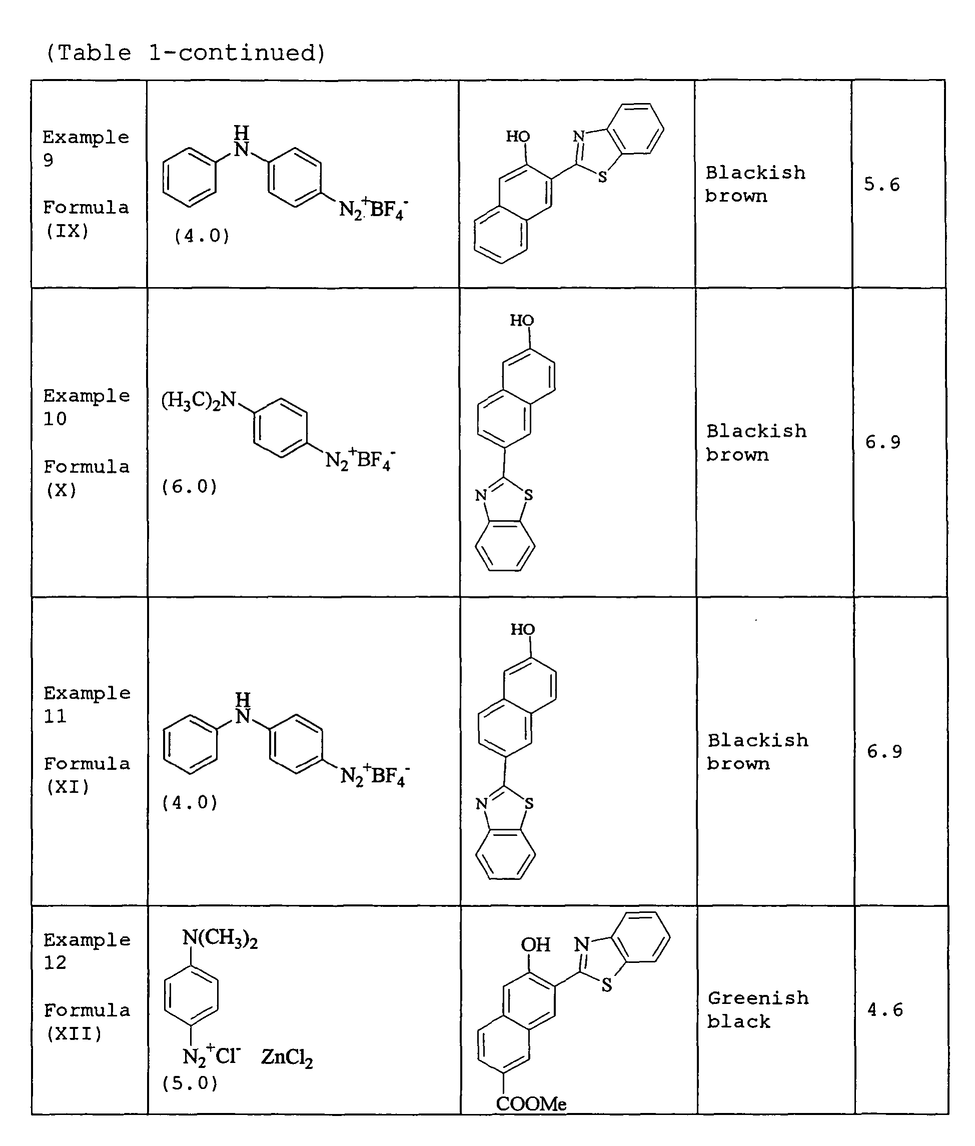

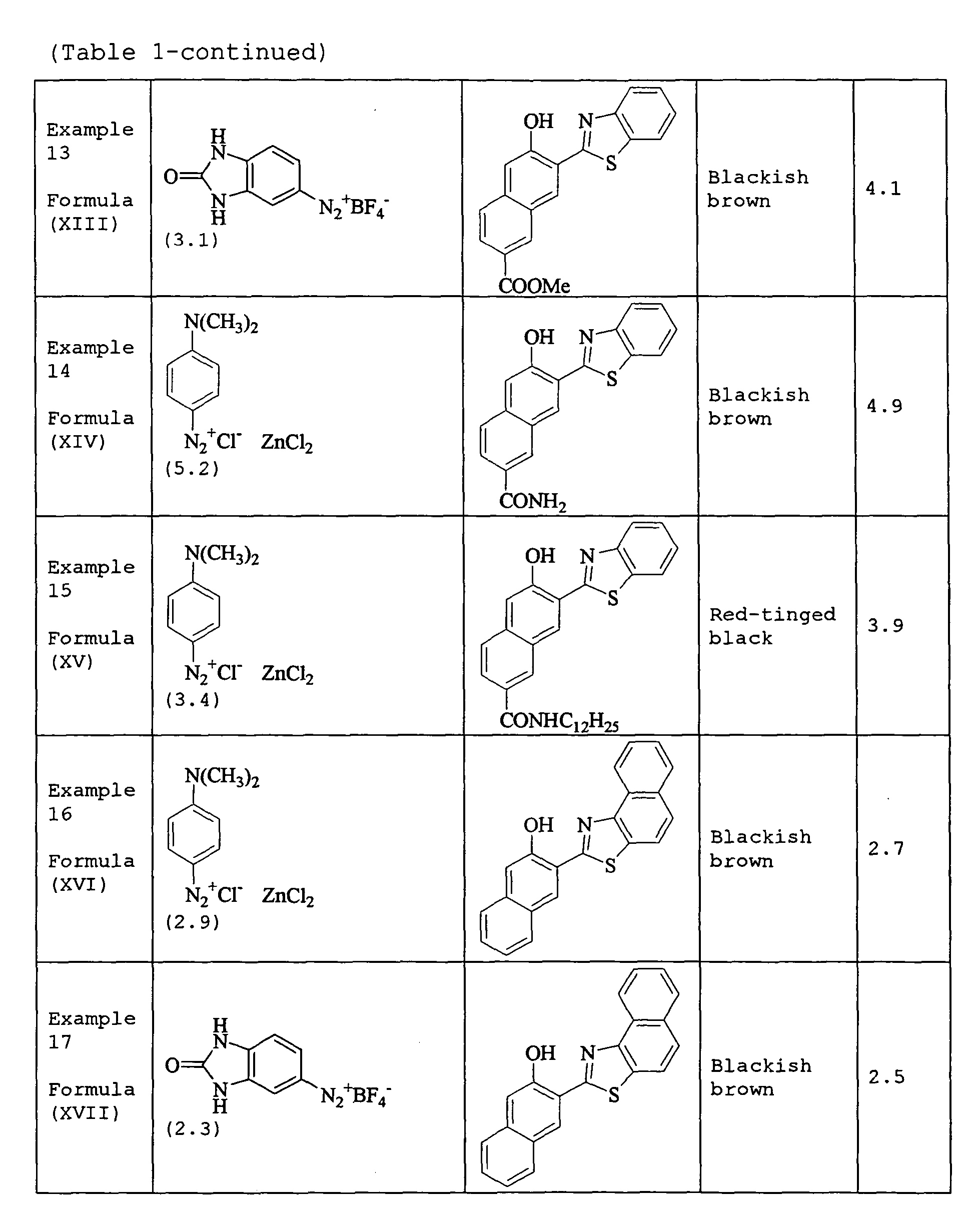

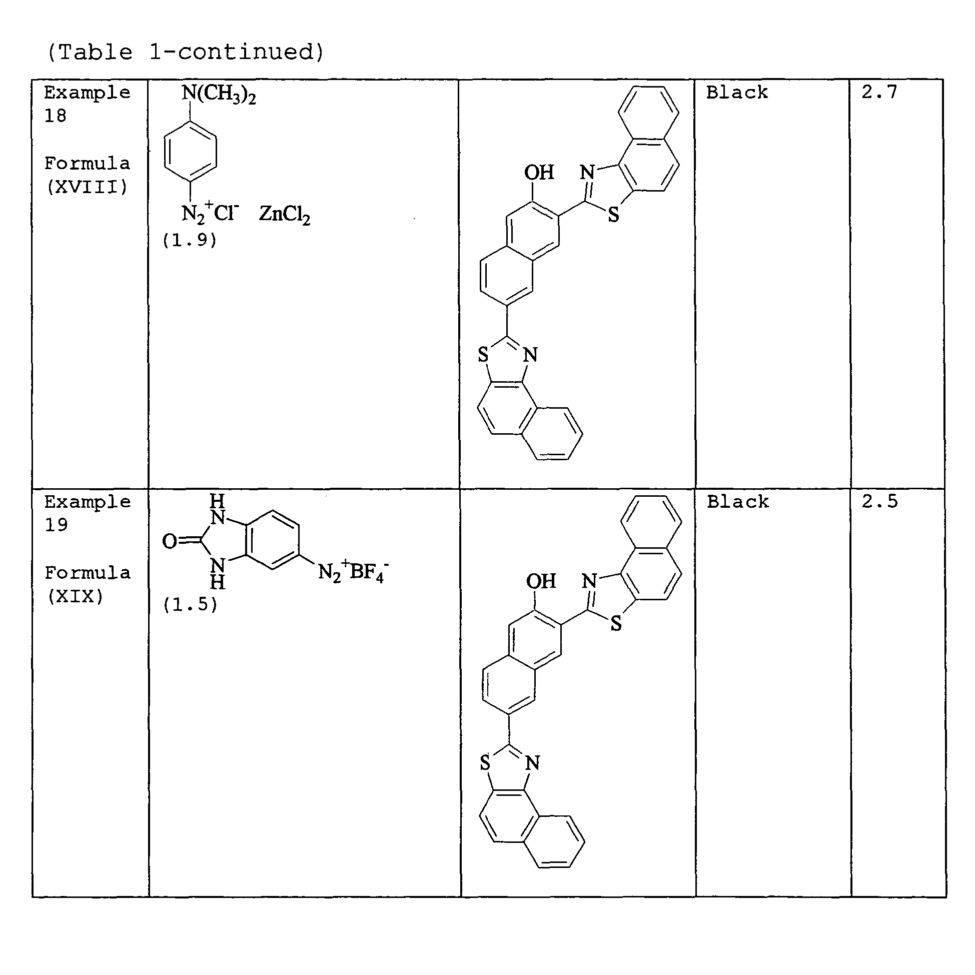

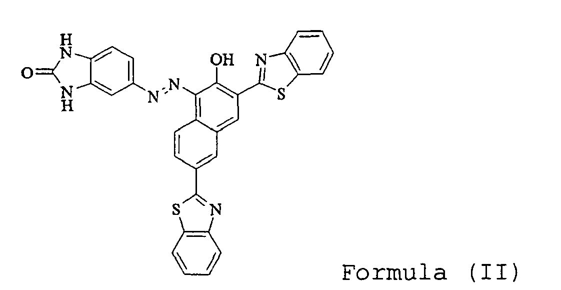

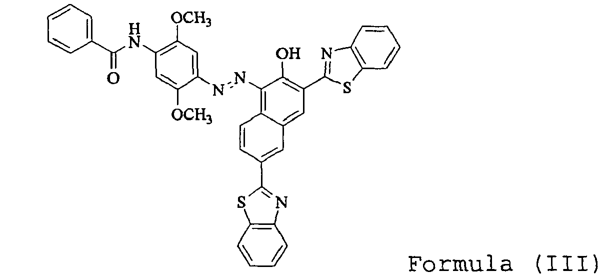

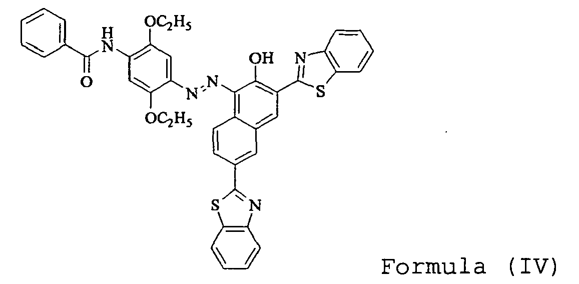

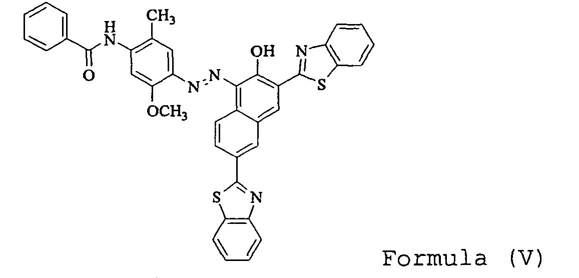

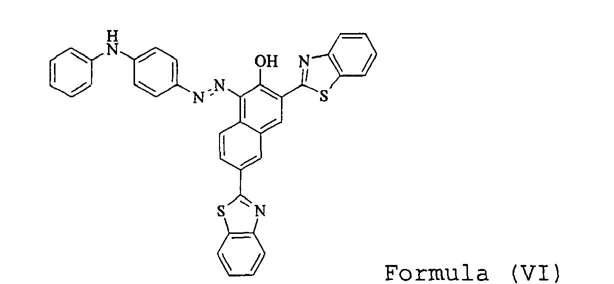

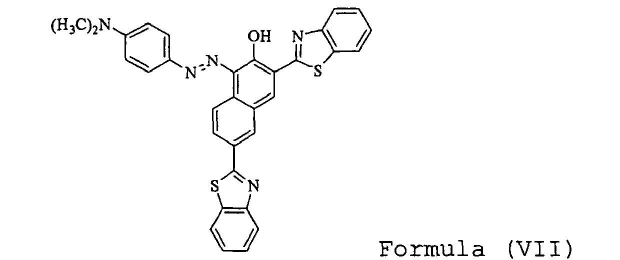

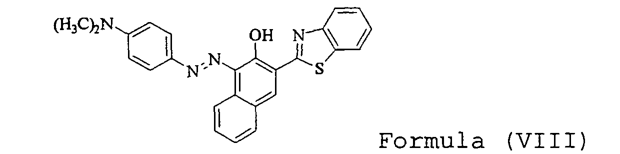

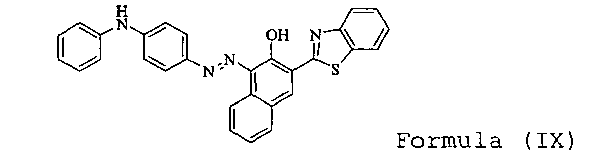

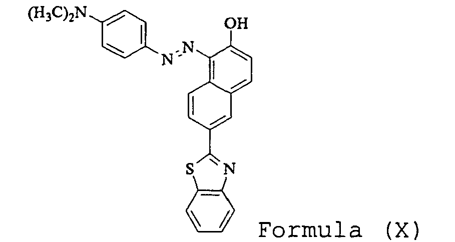

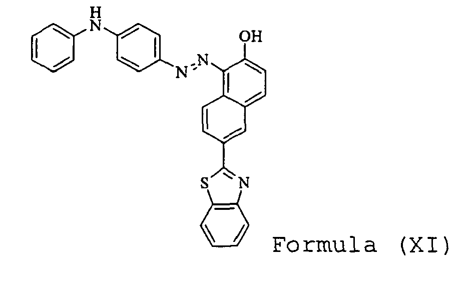

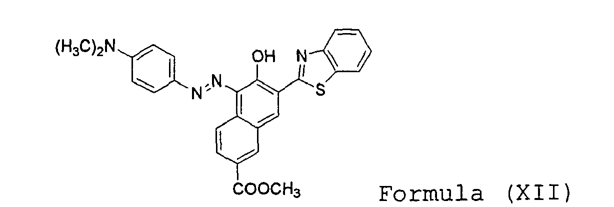

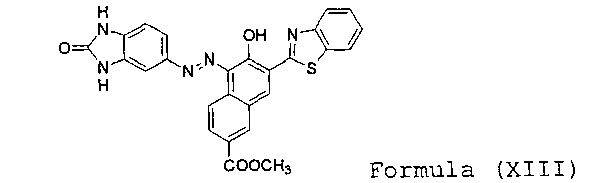

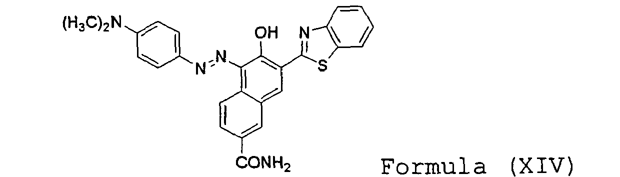

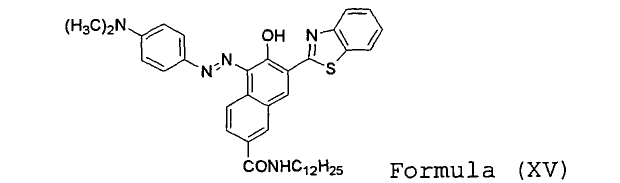

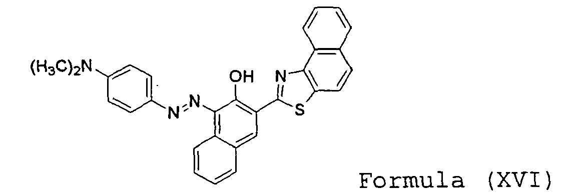

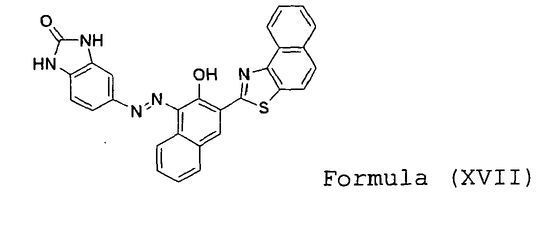

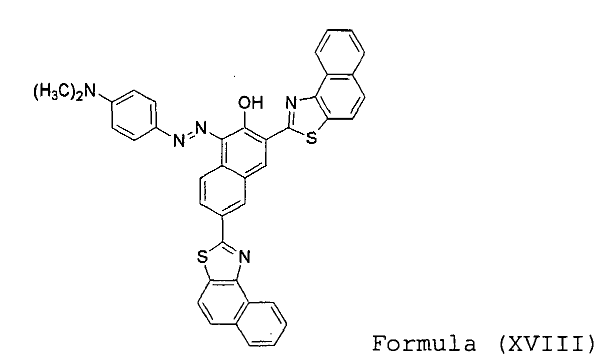

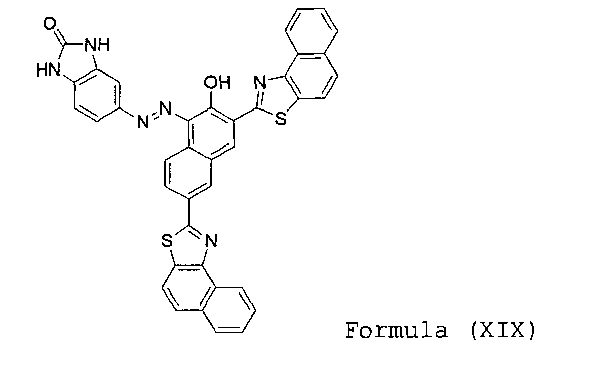

### Preparation of a black color paint

Materials shown in Table 2 were fed into a 50 cc mayonnaise bottle and the mixture was processed with dispersing shaker (DAS 200, LAU industries) for two hours to give a pigment dispersion. To this pigment dispersion, 7.93 g of alkyd resin varnish (shown in Table 2) and 3.87 g of melamine resin varnish (SUPER BECKAMINE G-821-60, DAINIPPON INK AND CHEMICALS, INCORPORATED) were admixed to give a paint.

As a black color compound, any one of the following was used:
.a pigment obtained by any one of Examples 2, 3, 4, 6, and 7;
**.Comparative example 1**: aniline black (PALIOGEN BLACK L 0080, BASF Aktiengesellschaft);
**.Comparative example 2**: carbon black (TOKABLACK # 8500, Tokai Carbon, Co. Ltd.).

**Table 2**

| | |
|---|---|
| Black color compound | 1.00 g |
| Alkyd resin varnish (BECKOSOL1323-60-EL, DAINIPPON INK AND CHEMICALS, INCORPORATED) | 3.67 g |
| Dispersant (BYK-161, BYK-Chemie GmbH) | 1.00 g |
| 7/3 Mixture of xylol /n-butanol (Kishida Chemical Co.,Ltd.) | 2.53 g |
| Glass beads (GB503M 1.5 mm diameter, Toshiba-Ballotini Co.,Ltd.) | 8.30 g |

### Evaluation of paint shielding properties

Paints obtained as above were applied on OHP film (A4 size 0.1 mm thick sheets, Uchida Yoko Co., Ltd.) with bar coater Rod No.44. The sheets were left dried at room temperature for 10 minutes and baked at 120°C for 25 minutes.

Thus obtained painted sheets were put on contrast ratio test papers (JIS K 5400, Taiyukizai.,Co.,Ltd.) and color differences between area of the sheet under which the color was black and that under which the color was white were measured with colorimeter (Macbeth color-eye 7000, viewing angle 2°, SAKATA INX CORPORATION) and then shielding properties were evaluated.

The result of evaluation of shielding properties is shown in Table 3.

**Table 3**

| Compound | Shielding property (ΔE*ab) |
|---|---|
| Example 2 | 0.24 |
| Example 3 | 0.14 |
| Example 4 | 0.21 |
| Example 6 | 0.06 |
| Example 7 | 0.01 |
| Comparative example 1 | 0.50 |
| Comparative example 2 | 0.60 |

The paints comprising the black color monoazo compounds of the present invention exhibited excellent shielding properties.

### Evaluation of weather resistances of paint film

The materials shown in Table 4 were fed in a 500 cc aluminum can and mixed with homodisper (TK-2.5, TOKUSYU KIKA KOGYO Co., Ltd.) for two hours to give a pigment dispersion. To this pigment dispersion, 26.6 g of alkyd resin varnish (Table 4) and 15.8 g of melamine resin varnish (SUPER BECKAMINE G-821-60, DAINIPPON INK AND CHEMICALS, INCORPORATED) were admixed to give a white paint.

### Table 4

| | |
|---|---|
| Rutile type titanium dioxide (TIPAQUE CR-97, ISHIHARA SANGYO Co.,Ltd.) | 20.0 g |
| Alkyd resin varnish (BECKOSOL 1323-60-EL, DAINIPPON INK AND CHEMICALS, INCORPORATED) | 20.9 g |
| 7/3 Mixture of xylol /n-butanol (Kishida Chemical Co.,Ltd.) | 2.53 g |
| Glass beads (GB 503M 1.5mm diameter, Toshiba-Ballotini Co.,Ltd.) | 200 g |

Five weight parts of the black color paint, which was prepared by the method described above and comprised the monoazo compound obtained in Example 2 or 7, and the 95 weight parts of the white paint were mixed to give a light-color paint.

The light-color paint obtained as above was applied on chromate treated aluminum plate (A5052P 0.5 mm thick, Taiyukizai. Co.,Ltd.) with bar coater Rod No.44. The plate was air dried for 10 minutes and baked at 120°C for 25 minutes to give light-color paint film.

Thus obtained light-color paint film was exposed to xenon light using Super Xenon Weather Meter (SX-75, SUGA TEST INSTRUMENTS Co., Ltd.) under the test condition shown below to conduct the weather resistance test. After the exposure for 100 hours, chromatic coordinates were measured with colorimeter (Macbeth color-eye 7000, viewing angle 2°, SAKATA INX CORPORATION) and the color differences ΔE*_{ab} between before and after the exposure were evaluated.

According to the same method as above, a light-color paint film comprising aniline black (PALIOGEN BLACK L 0080, BASF Aktiengesellschaft) was prepared as Comparative example 1 and the weather resistance of the same was evaluated. The result of evaluation of weather resistances of light-color paint film is shown in Table 5.

### Exposure condition

The exposure condition was as follows;
Exposure energy: 180 W/m²
Exposure time: one hour and forty-two minutes (black panel temperature 63°C, relative humidity 50%)
Rainfall time: 18 minutes (dry-bulb temperature 28°C, relative humidity 95%)

**Table 5**

| Compound | Weather resistance (ΔE*ab) |
|---|---|
| Example 2 | 1.4 |
| Example 7 | 1.7 |
| Comparative example 1 | 2.7 |

The light-color paint film comprising the black color monoazo compounds of the present invention showed low degrees of color changes and exhibited excellent weather resistances.

### Preparation of ink

The materials shown in Table 6 were fed in a 50 cc mayonnaise bottle and processed with dispersing shaker (DAS200, LAU industries) for three hours to give a pigment dispersion. Paints obtained as above were applied on OHP film (A4 size and 0.1 mm thick sheets, Uchida Yoko Co., Ltd.) with bar coater Rod No.44. The film was air dried to give ink paint film.

**Table 6**

| | |
|---|---|
| Black color compound | 0.50 g |
| Nitrocellulose (H1/2) (Kishida Chemical Co.,Ltd.) | 1.425 g |
| Dioctyl adipate (Kishida Chemical Co.,Ltd.) | 0.285 g |
| Methyl cellosolve (Kishida Chemical Co.,Ltd.) | 0.760 g |
| Methylisobutylketone (Kishida Chemical Co.,Ltd.) | 2.47 g |
| 2-Propanol (Kishida Chemical Co.,Ltd.) | 4.56 g |
| Glass beads (GB 503M, 1.5 mm diameter, Toshiba-Ballotini Co.,Ltd.) | 18.0 g |

As black color compounds, the monoazo compounds obtained in Examples 1, 2, 6 and 7, aniline black (Comparative example 1)(PALIOGEN BLACK L 0080, BASF Aktiengesellschaft), and carbon black (Comparative example 2) (TOKABLACK #8500, Tokai Carbon, Co. Ltd.) were used.

The ink paint film were exposed to xenon light (SUNTEST CPS+, Atlas Material Testing Technology LLC) under the condition of black standard temperature of 45°C and exposure energy of 756 w/m². After 100 hours exposure, the color changes were evaluated by visual observation using gray-scale for evaluation of discoloration (JIS L 0804). The result is shown in Table 7.

**Table 7**

| Compound | Light resistance (gray-scale) |
|---|---|
| Example 1 | 4 |
| Example 2 | 4-5 |
| Example 6 | 5 |
| Example 7 | 4-5 |
| Comparative example 1 | 3-4 |
| Comparative example 2 | 3-4 |

The ink paint film comprising the black color monoazo compounds of the present invention showed low degrees of color changes caused by the exposure to xenon light and exhibited excellent light resistances.

### Evaluation of heat resistances

**Table 8**

| | |
|---|---|
| Black color compound | 0.1 g |
| Magnesium stearate (special grade chemicals, Wako Pure Chemical Industries,Ltd.) | 0.1 g |
| Polypropylene (Grandpolypro J707LA, Grand Polymer Co.) | 99.8 g |

As black color compounds, the monoazo compounds obtained in Examples 1, 2, 6 and 7, and aniline black (PALIOGEN BLACK L 0080, BASF Aktiengesellschaft), which served as Comparative example 1, were used.

The materials shown in Table 8 containing each of black color compounds were fed in a 1L polybottle and the mixture was shaken with shaker (Turbula mixer, Taiyukizai, Co., Ltd.) for 12 minutes to give a dispersion. The resulting polypropylene composition was molded at 200°C by using an injection molding machine (TUPARL TR-80S, Sodick Plustech Co., Ltd.) to give a test specimen. In addition, the composition was retained in the same injection molding machine at a temperature of 260°C for 5 minutes to give a test specimen. The chromatic coordinates of test specimens molded at temperatures of 200°C and 260°C were measured with colorimeter (Macbeth color-eye 7000, viewing angle 2°, SAKATA INX CORPORATION) and color differences ΔE^{*}ab between them were evaluated. The result is shown in Table 9.

**Table 9**

| Compound | Heat resistance (ΔE*_{ab}) |
|---|---|
| Example 1 | 1.1 |
| Example 2 | 1.0 |
| Example 6 | 1.2 |
| Example 7 | 0.6 |
| Comparative example 1 | 1.6 |

The test specimens pigmentized with the black color monoazo compounds of the present invention which were retained in injection molding machine at a temperature of 260°C showed low degrees of color changes compared to those molded at a temperature of 200°C. The specimens comprising the monoazo compounds of the present invention exhibited excellent heat resistances.

### Manufacture of black matrix

As black color compounds, monoazo compounds obtained in Examples 2, 6 and 7 were used. Aniline black (PALIOGEN BLACK L 0080, BASF Aktiengesellschaft) was also used as the black color compound of Comparative example 1. Materials shown in Table 10 were fed in a 50 cc mayonnaise bottle and the mixture was processed with dispersing shaker (DAS200, LAU industries) for six hours to give a pigment dispersion. To the resulting pigment dispersion, materials shown in Table 11 were added and the mixture was processed with the same shaker as above for 30 minutes to give a black color ink composition which was used for the photolithographic method.

**Table 10**

| | |
|---|---|
| Black color compound | 0.6 g |
| 1,2-propanediol 1-monomethylether 2-acetate (Tokyo Kasei Kogyo Co., Ltd.) | 5.0 g |
| Zirconia beads (0.3 mm diameter) | 10.0 g |

**Table 11**

| | |
|---|---|
| Photosensitive resin (Cyclomer P200, Daicel Chemical Industries, Ltd.) | 2.5 g |
| Pentaerythritol tetraacrylate (Sigma-Aldrich Co.) | 0.2 g |
| 2-Benzyl-2-dimethylamino-4'-morpholinobutyrophenone (Sigma-Aldrich Co.) | 0.05 g |
| 2,4-Diethyl-9H-thioxanthen-9-one (Tokyo Kasei Kogyo Co., Ltd.) | 0.05 g |
| 1,2-Propanediol 1-monomethylether 2-acetate (Tokyo Kasei Kogyo Co., Ltd.) | 0.8 g |
| Cyclohexanone (Tokyo Kasei Kogyo Co., Ltd.) | 0.2 g |

Thus obtained black color ink composition was applied on a slide glass using bar coater Rod No.10 and the slide glass was dried in an 80°C oven for five minutes to give an ink paint film. A portion of the paint film was masked and the film was exposed to light using a high-pressure mercury vapor lamp at a condition of 200 mJ/cm². Thereafter, the film was developed with a 0.5% aqueous sodium acid carbonate at a temperature of 25°C and then dried in a 220°C oven for 20 minutes to give a black matrix.

Thus obtained black matrices were put on contrast ratio test papers (JIS K 5400, Taiyukizai.,Co.,Ltd.) and color differences between portions of film under which the color was black and those under which the color was white were measured with colorimeter (Macbeth color-eye 7000, viewing angle 2°, SAKATA INX CORPORATION) and then shielding properties were evaluated.

The result of evaluation of shielding properties is shown in Table 12.

**Table 12**

| | Shielding property (ΔE*_{ab}) |
|---|---|
| Example 2 | 0.01 |
| Example 6 | 0.05 |
| Example 7 | 0.02 |
| Comparative example 1 | 1.42 |

The black matrices manufactured by using the resist inks comprising the black color monoazo compounds of the present invention exhibited excellent shielding properties. In addition, the black matrices comprising the compounds of the present invention did not show any light deterioration, for example, discoloration after the exposure to light irradiated by the high-pressure mercury vapor lamp.

### Industrial Applicability

The monoazo compound of the present invention can be suitably used for pigments such as those contained in electrophotographic toners, pigment solid solutions, inks for jet ink printings, aqueous or oily print inks for common printings, aqueous or oily paints for automobiles and building materials, dies for dying cellulose fibers, and resist inks used for manufacture of black matrices applied to color filters for displays.

## Claims

1. A monoazo compound represented by formula (1): 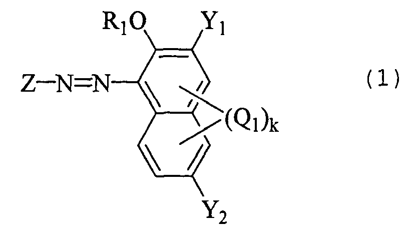 wherein Y₁ and Y₂ are selected from the group consisting of hydrogen atom, a group represented by formula (2) and a group represented by formula (3), provided that at least one of Y₁ and Y₂ represents a group represented by formula (2); 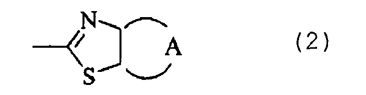
-CO-E-X (3)
wherein A is an optionally substituted aromatic group or an optionally substituted heterocyclic group having conjugated double bonds;
E is selected from -O- and -NH-;
X is selected from the group consisting of hydrogen atom, an optionally branched and optionally substituted, saturated or unsaturated aliphatic group having 1-20 carbon atoms, an optionally substituted aromatic group and an optionally substituted heterocyclic group having conjugated double bonds;
Q₁ is a group selected from the group consisting of an optionally branched lower alkyl group, an optionally branched lower alkoxy group, halogen atom, nitro group and nitroso group;
k is an integer of 0-3;
R₁ is selected from the group consisting of hydrogen atom, an alkaline metal, an optionally substituted and optionally branched alkyl group having 1-20 carbon atoms, an optionally substituted and optionally branched acyl group having 1-20 carbon atoms and phenylalkyl group;
Z is selected from the group consisting of a group represented by formula (4), a group represented by formula (5) and a group represented by formula (6); 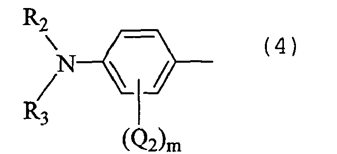 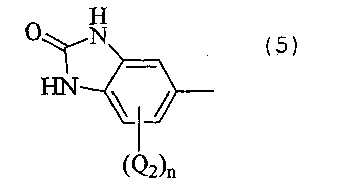 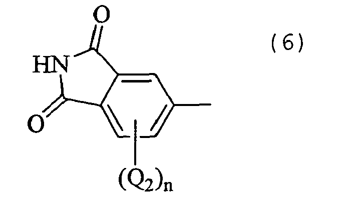 wherein Q₂ is selected from the group consisting of halogen atom, halogenated lower alkyl group, nitro group, lower alkyl group, lower alkoxy group, cyano group, phenoxy group, amino group, pyrimidylamino group, benzoylamino group, sulfonic group, hydroxy group, lower alkylaminosulfonyl group, lower alkoxycarbonyl group, phenoxycarbonyl group, lower alkylaminocarbonyl group, phenylaminocarbonyl group and alkenyl group having 2-6 carbon atoms; in which when Q₂ has an aromatic group, said aromatic group may have further substituent(s) selected from halogen atom, lower alkyl group, lower alkoxy group, phenyl group and cyano group;
m is an integer of 0-4;
n is an integer of 0-3;
R₂ and R₃ may be different or the same and are selected from the group consisting of hydrogen atom, an optionally branched and optionally substituted lower alkyl group, lower aliphatic acyl group, an optionally substituted benzoyl group and an optionally substituted phenyl group; in which when R₂ or R₃ has an aromatic group, said aromatic group may have further substituent (s) selected from halogen atom, lower alkyl group, lower alkoxy group, phenyl group and cyano group;
or a salt thereof.

2. A process for preparing the monoazo compound of claim 1, comprising coupling a coupler represented by formula (7) with a diazonium salt obtained by diazotizing an aniline compound selected from formula (8), formula (9) and formula (10): 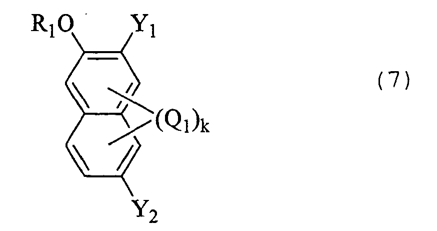 wherein Y₁, Y₂, Q₁, k and R₁ are the same as defined in claim 1; 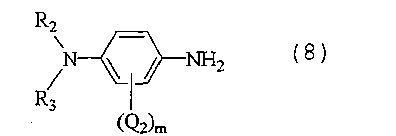 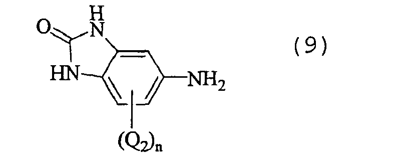 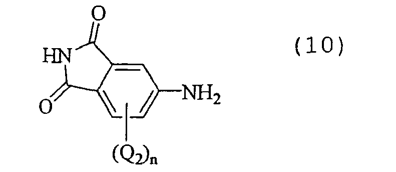 wherein Q₂, m, n, R₂ and R₃ are the same as defined in claim 1.

3. A method for purifying the monoazo compound or salt thereof of claim 1, comprising suspending the monoazo compound or salt thereof of claim 1 in an organic solvent selected from alcohols and aprotic polar solvents and stirring the suspension at temperatures of 70-150°C.

4. The monoazo compound or salt thereof which is purified by the method of claim 3.

5. A pigment comprising the monoazo compound of claim 1.

6. A print ink comprising the monoazo compound of claim 1.

7. A paint comprising the monoazo compound of claim 1.

8. A dye comprising the monoazo compound of claim 1.

9. A resist ink comprising the monoazo compound of claim 1.

10. A black matrix used for a display which is made of the resist ink of claim 9.
